# EUROPEAN PATENT SPECIFICATION

(11) **EP 3 873 488 B1**
(45) Date of publication and mention of the grant of the patent: **19.03.2025**
(21) Application number: 19878475.3
(22) Date of filing: 24.10.2019
(51) Int. Cl.: A61K 31/713, A61K 48/00, A61P 1/16, A61P 31/20, A61K 38/21, C12Q 1/6883

(54) **PHARMACEUTICAL COMPOSITIONS FOR USE IN TREATING AN HBV OR HDV INFECTION**
PHARMAZEUTISCHE ZUSAMMENSETZUNGEN ZUR VERWENDUNG BEI DER BEHANDLUNG EINER HBV- ODER HDV-INFEKTION
COMPOSITIONS PHARMACEUTIQUES POUR LE TRAITEMENT D'UNE INFECTION PAR LE VHB OU LE VHD

(30) Priority: 31.10.2018 AU 2018904123
(43) Date of publication of application: 08.09.2021
(73) Proprietor: The University of Sydney, New South Wales 2006 (AU); The Westmead Institute For Medical Research, Westmead, New South Wales 2145 (AU); Western Sydney Local Health District, Westmead, New South Wales 2145 (AU)
(72) Inventor: DOUGLAS, Mark William, SYDNEY New South Wales 2006 (AU); ESLAM, Mohammed, SYDNEY New South Wales 2006 (AU); JACOB, George, SYDNEY New South Wales 2006 (AU); KHAN, Anis, SYDNEY New South Wales 2006 (AU)
(74) Representative: K&L Gates LLP
(86) International application number: PCT/AU2019/051169
(87) International publication number: WO 2020/087107

(56) References cited:
- WO-A1-2011/003071
- WO-A1-2013/061295
- WO-A1-2014/094645
- WO-A1-2016/054421
- WO-A1-2016/176745
- CHONG TEIK LIM ET AL: "Hepatitis B and concomitant hepatic steatosis", JOURNAL OF THORACIC DISEASE, vol. 5, no. 3, 1 February 2017 (2017-02-01), China, pages 1 - 5, XP055706931, ISSN: 2072-1439, DOI: 10.21037/atm.2016.12.04
- JULIA KOZLITINA, ERIKS SMAGRIS, STEFAN STENDER, BØRGE G NORDESTGAARD, HEATHER H ZHOU, ANNE TYBJÆRG-HANSEN, THOMAS F VOGT, HELEN H : "Exome-wide association study identifies a TM6SF2 variant that confers susceptibility to nonalcoholic fatty liver disease", NATURE GENETICS, vol. 46, no. 4, 1 April 2014 (2014-04-01), pages 352 - 356, XP055706921, ISSN: 1061-4036, DOI: 10.1038/ng.2901
- H. MAHDESSIAN, A. TAXIARCHIS, S. POPOV, A. SILVEIRA, A. FRANCO-CERECEDA, A. HAMSTEN, P. ERIKSSON, F. VAN'T HOOFT: "TM6SF2 is a regulator of liver fat metabolism influencing triglyceride secretion and hepatic lipid droplet content", PROCEEDINGS OF THE NATIONAL ACADEMY OF SCIENCES, vol. 111, no. 24, 17 June 2014 (2014-06-17), pages 8913 - 8918, XP055706923, ISSN: 0027-8424, DOI: 10.1073/pnas.1323785111
- YAQI WANG, TING WU, DANQING HU, XINXIN WENG, XIAOJING WANG, PEI-JER CHEN, XIAOPING LUO, HONGWU WANG, QIN NING: "Intracellular hepatitis B virus increases hepatic cholesterol deposition in alcoholic fatty liver via hepatitis B core protein", JOURNAL OF LIPID RESEARCH , vol. 59, no. 1, 1 January 2018 (2018-01-01), pages 58 - 68, XP055706927, ISSN: 0022-2275, DOI: 10.1194/jlr.M079533
- MANGIA ALESSANDRA, BERG THOMAS, LIK HENRY, CHAN YUEN, IRVING WILLIAM L, DORE GREGORY J, MARIA LORENA, ABATE, BUGIANESI ELISABETTA,: "Diverse impacts of the rs58542926 E167K variant in TM6SF2 on Viral and metabolic liver disease phenotypes", HEPATOLOGY , vol. 64, no. 1, 28 January 2016 (2016-01-28), pages 34 - 46, XP055806057, DOI: 10.1002/hep.28475
- YI-XIAN SHI, HUANG CHEN-JIE, YANG ZHENG-GANG: "Impact of hepatitis B virus infection on hepatic metabolic signaling pathway", WORLD JOURNAL OF GASTROENTEROLOGY, vol. 22, no. 36, 1 January 2016 (2016-01-01), pages 8161 - 8167, XP055706929, ISSN: 1007-9327, DOI: 10.3748/wjg.v22.i36.8161
- HU DANQING; WANG HONGWU; WANG HAI; WANG YAQI; WAN XIAOYANG; YAN WEIMING; LUO XIAOPING; NING QIN: "Non-alcoholic hepatic steatosis attenuates hepatitis B virus replication in an HBV-immunocompetent mouse model", HEPATOLOGY INTERNATIONAL, vol. 12, no. 5, 5 July 2018 (2018-07-05), pages 438 - 446, XP036617482, ISSN: 1936-0533, DOI: 10.1007/s12072-018-9877-7
- CHONG TEIK LIM, KUMAR RAJNEESH: "Hepatitis B and concomitant hepatic steatosis", ANNALS OF TRANSLATIONS MEDICINE , vol. 5, no. 3, 38, 1 February 2017 (2017-02-01), pages 1 - 5, XP055706931, ISSN: 2072-1439, DOI: 10.21037/atm.2016.12.04
- ZHANG XIAOYU, LIU SHOUSHENG, DONG QUANJIANG, XIN YONGNING, XUAN SHIYING: "The genetics of clinical liver diseases: Insight into the TM6SF2 E167K variant", JOURNAL OF CLINICAL AND TRANSLATIONAL HEPATOLOGY, vol. 6, no. 3, 7 September 2018 (2018-09-07), pages 326 - 331, XP055806059, ISSN: 2225-0719, DOI: 10.14218/JCTH.2018.00022
- CHENG YUAN-LUNG, WANG YUAN-JEN, KAO WEI-YU, CHEN PING-HSIEN, HUO TEH-IA, HUANG YI-HSIANG, LAN KENG-HSIN, SU CHIEN-WEI, CHAN WAN-LE: "Inverse association between Hepatitis B virus infection and fatty liver disease: A large-scale study in populations seeking for check-up", PLOS ONE, vol. 8, no. 8, e72049, 22 August 2013 (2013-08-22), pages 1 - 9, XP055806063, ISSN: 1932-6203, DOI: 10.1371/journal.pone.0072049
- WANG LU; WANG YIJIN; LIU SHUHONG; ZHAI XIANGWEI; ZHOU GUANGDE; LU FENGMIN; ZHAO JINGMIN: "Nonalcoholic fatty liver disease is associated with lower hepatitis B viral load and antiviral response in pediatric population", JOURNAL OF GASTROENTERLOGY, vol. 54, no. 12, 27 May 2019 (2019-05-27), pages 1096 - 1105, XP036941923, ISSN: 0944-1174, DOI: 10.1007/s00535-019-01594-6
- XINPEI CHEN, PENGCHENG ZHOU, LUO DE, BO LI, SONG SU: "The roles of transmembrane 6 superfamily member 2 rs58542926 polymorphism in chronic liver disease: A meta-analysis of 24,147 subjects", MOLECULAR GENETICS & GENOMIC MEDICINE, vol. 7, e827, 1 August 2019 (2019-08-01), pages 1 - 8, XP055706932, ISSN: 2324-9269, DOI: 10.1002/mgg3.824

## Description

### Field of the disclosure

The present disclosure relates to compositions and methods for treating viral infections and in particular for treating hepatitis B or hepatitis D viral infections.

### Background of the disclosure

Any discussion of the prior art throughout the specification should in no way be considered as an admission that such prior art is widely known or forms part of the common general knowledge in the field.

Hepatitis B is a viral disease and a major cause of cirrhosis and mortality worldwide. The disease is caused by the hepatitis B virus (HBV) which can be transmitted by blood, bodily fluids and from mother to child.

The HBV viral particle comprises a lipid envelope, decorated with hepatitis B surface antigen (HBsAg), which surrounds the viral core. The core comprises a protein shell known as a capsid, which in turn contains the virus' partially double-stranded, circular DNA genome as well as viral and host proteins. Following infection of a hepatocyte, the HBV genome is delivered into the nucleus and repaired to form a mini chromosome known as a covalently closed circular DNA (cccDNA) which acts as a template for the transcription of viral RNAs. The viral RNAs are translated by host cellular machinery to produce viral proteins. Full length viral RNA molecules known as pre-genomic RNA (pgRNA) interact with viral capsid proteins, including core antigen (HBcAg), and polymerase to form capsid particles. Within the confines of the capsid, the polymerase reverse transcribes the pgRNA and thus replicates the circular DNA genome. Once a near full length circular DNA is formed by reverse transcription of the viral pgRNA, an immature capsid becomes a mature capsid. The encapsidated viral genome then typically associates with cellular lipids and viral envelope proteins (S-HBsAg, M-HBsAg and L-HBsAg) to form virions which may be secreted.

However, non-infectious subviral particles (SVPs) are also produced, and indeed most of the HBsAg in the blood of an infected individual is in the form of non-infectious particles which contain no viral DNA. These non-infectious particles are particularly relevant from a clinical standpoint as HBsAg can suppress both the adaptive and innate immune responses against the virus (Op den Brouw et al. (2009) Immunology 126: 280-289; Wu et al. (2009) Hepatology 49: 1132-1140).

Co-infection with hepatitis D virus (HDV or delta virus) accelerates the progression of liver disease caused by HBV. Small delta hepatitis D antigen (S-HDAg) and large delta hepatitis D antigen (L-HDAg) are two forms of the only protein encoded by the HDV genome (Sureau and Negro (2016) J Heptaol64, S102-S116). S-HDAg is required for viral replication and L-HDAg for vial assembly via interactions with HBsAg (Sureau and Negro (2016) J Heptaol 64, S102-S116). HDV virions are secreted via the ER-Golgi pathway, rather than via the multi-vesicular body/Endosomal Sorting Complexes Required for Transport (ESCRT) pathway which is used by HBV (Cornberg et al. (2017) J Hepatol 66, 398-411).

Much of the research into hepatitis B treatments has aimed at developing a so called "functional cure", which generally refers to the sustained loss of HBsAg from blood (Lok et al. (2017) Hepatology 66, 1296-1313). However, current therapies have provided low rates of HBsAg clearance, comparable to those observed with placebos (Janssen et al. (2005) Lancet 365, 123-129; Marcellin et al. (2004) N Engl J Med 351, 1206-1217; Buster et al. (2007) Hepatology 46, 388-394).

Current HBV therapeutics are generally limited to interferons and nucleoside analogues. Interferons are generally less effective at suppressing viral replication compared to nucleoside analogues. Moreover, they require parenteral administration, are associated with several adverse effects and are contraindicated in patients with decompensated cirrhosis or severe exacerbations of hepatitis and patients with autoimmune or psychiatric illnesses (Lok et al. (2017) Journal of Hepatology 67, 847-861). Nucleoside analogues such as lamivudine, entecavir, tenofovir and telbivudine suppress HBV replication by targeting its polymerase enzyme. However, although those inhibitors retard disease progression, they do not remove HBV from the liver, and viremia and disease progression usually rebound once therapy is stopped (Terrault et al. (2016) Hepatology 63, 261-283; Chang et al. (2016) N Eng J Med354, 1001-1010). Long term treatment with these inhibitors is usually required, increasing the cost of treatment and the risk of nonadherence and adverse effects (Lok et al. (2017) Journal of Hepatology 67, 847-861).

In 2017, the Hepatitis B Foundation asked 30 experts in the fields of hepatitis B research and liver cancer to identify areas of research which they considered important to the goal of finding a cure for chronic hepatitis B and D (Block et al. (2018) Antiviral Research 150, 93-100). Among the areas identified as "top priorities" was the repression of HBV cccDNA by elimination or transcriptional repression. cccDNA acts as a template for the transcription of pgRNA and therefore plays an important role in the virus's life cycle (Seeger and Mason (2015) Virology 479-480C, 672-686). Also identified as a top priority was the development of antiviral therapeutics targeting hepatitis Bx protein (HBx) and HBsAg (Block et al. (2018) Antiviral Research 150, 93-100). Although the function of HBx remains unclear, it appears to be essential for replication of the virus *in vivo* (Seeger and Mason (2015) Virology 479-480C, 672-686). Other areas of research that were considered important included the development of antivirals targeting hepatitis B core antigen (HBcAg) and hepatitis B e antigen (HBeAg) (Block et al. (2018) Antiviral Research 150, 93-100). Although much research is being conducted in these areas, the available options for treating HBV infection remain limited. In this context, there is a need for compositions and methods for treating hepatitis B.

### Summary of the disclosure

The present disclosure relates to the surprising identification of an endogenous target for treating hepatitis B and hepatitis D. The present inventors have surprisingly found that HBV and HDV infections can be treated by inhibiting a regulator of liver lipid metabolism in a patient. In work leading to the present disclosure, the inventors have found that HBsAg secretion and HDV replication can be suppressed by inhibiting the activity of transmembrane 6 superfamily member 2 (TM6SF2), a protein with known roles in regulating liver lipid metabolism.

Accordingly, in one aspect, the present disclosure provides a method of treating a hepatitis B virus infection in a subject the method comprising administering to the subject an inhibitor wherein the inhibitor inhibits or suppresses a regulator of liver lipid metabolism in the subject. The present disclosure also provides an inhibitor for use in a method of treating a hepatitis B virus infection in a subject, wherein the inhibitor inhibits or suppresses a regulator of liver lipid metabolism in the subject. The regulator may regulate lipid synthesis and/or lipid secretion in the liver. The regulator may further regulate cholesterol synthesis and/or lipoprotein secretion in the liver.

The invention uses an inhibitor of TM6SF2.

The inhibitor may be selected from the group consisting of an antibody or a fragment thereof, a nucleic acid and a small organic molecule.

Preferably, the inhibitor reduces HBsAg concentration in serum of the subject.

In certain embodiments, the inhibitor is a nucleic acid or a small organic molecule.

The nucleic acid preferably reduces accumulation of TM6SF2 mRNA in the subject.

In certain embodiments, the nucleic acid comprises at least 20 contiguous nucleotides which are substantially complementary to the nucleotide sequence set forth in SEQ ID NO. 3.

In certain embodiments, the nucleic acid comprises at least 10 contiguous nucleotides differing by no more than 3 nucleotides from a nucleotide sequence set forth in any one of SEQ ID NOs. 1, 2 or 4. For example, the nucleic acid may comprise at least 10 contiguous nucleotides differing by no more than 3 nucleotides from a nucleotide sequence set forth in SEQ ID NO. 4. For example, the nucleic acid may comprise at least 20 contiguous nucleotides differing by no more than 3 nucleotides from a nucleotide sequence set forth in SEQ ID NO. 4.

In certain embodiments, the nucleic acid comprises at least 10 contiguous nucleotides which are at least 80% identical to a nucleotide sequence set forth in any one of SEQ ID NOs 1, 2 or 4. For example, the nucleic acid may comprise at least 10 contiguous nucleotides which are at least 80% identical to a nucleotide sequence set forth in SEQ ID NO 4. For example, the nucleic acid may comprise at least 20 contiguous nucleotides which are at least 80% identical to a nucleotide sequence set forth in SEQ ID NO 4.

The nucleic acid may comprise the sequence set forth in SEQ ID NO. 5 or SEQ ID NO. 6 and a sequence substantially complementary thereto.

In certain embodiments, the nucleic acid is between 15 and 50 nucleotides in length.

The nucleic acid may be a siRNA or a nucleic acid encoding a siRNA. The siRNA may be conjugated to N-acetylgalactosamine.

In certain embodiments, the method further comprises administering to the subject an antiviral agent. The antiviral agent may be an interferon or a nucleoside analogue, or a prodrug thereof. For example, the antiviral agent may be pegylated interferon.

In some embodiments, the antiviral agent and the inhibitor are administered to the subject in separate compositions; or in combined compositions. In some embodiments, the antiviral agent and the inhibitor are administered to the subject sequentially or simultaneously.

The subject may be suffering from acute hepatitis B virus infection. The subject may be suffering from chronic hepatitis B virus infection.

The subject may also be suffering from a hepatitis D virus infection.

Preferably, the subject is a human.

In a second aspect, the present disclosure provides a method of reducing the concentration of hepatitis B surface antigen in serum of a subject the method comprising administering to the subject an inhibitor wherein the inhibitor inhibits or suppresses a regulator of liver lipid metabolism in the subject. The present disclosure also provides an inhibitor for use in a method of reducing the concentration of hepatitis B surface antigen in serum of a subject, the method comprising administering to the subject an inhibitor wherein the inhibitor inhibits or suppresses a regulator of liver lipid metabolism in the subject. The regulator may regulate lipid synthesis and/or lipid secretion in the liver. The regulator may further regulate cholesterol synthesis and/or lipoprotein secretion in the liver.

Preferably, the regulator is TM6SF2.

The inhibitor may be selected from the group consisting of an antibody or a fragment thereof, a nucleic acid and a small organic molecule. In certain embodiments, the inhibitor is a nucleic acid.

Preferably, the nucleic acid reduces accumulation of TM6SF2 mRNA in the subject.

In some embodiments, the nucleic acid comprises at least 20 contiguous nucleotides which are substantially complementary to the nucleotide sequence set forth in SEQ ID NO. 3.

In certain embodiments, the nucleic acid comprises at least 10 contiguous nucleotides differing by no more than 3 nucleotides from a nucleotide sequence set forth in any one of SEQ ID NOs. 1, 2 or 4. For example, the nucleic acid may comprise at least 10 contiguous nucleotides differing by no more than 3 nucleotides from a nucleotide sequence set forth in SEQ ID NO. 4. For example, the nucleic acid may comprise at least 20 contiguous nucleotides differing by no more than 3 nucleotides from a nucleotide sequence set forth in SEQ ID NO. 4.

In certain embodiments, the nucleic acid comprises at least 10 contiguous nucleotides which are at least 80% identical to a nucleotide sequence set forth in any one of SEQ ID NOs 1, 2 or 4. For example, the nucleic acid may comprise at least 10 contiguous nucleotides which are at least 80% identical to a nucleotide sequence set forth in SEQ ID NO 4. For example, the nucleic acid may comprise at least 20 contiguous nucleotides which are at least 80% identical to a nucleotide sequence set forth in SEQ ID NO 4.

In certain embodiments, the nucleic acid comprises the sequence set forth in SEQ ID NO. 5 or SEQ ID NO. 6 and a sequence substantially complementary thereto.

The nucleic acid may be between 15 and 50 nucleotides in length.

In certain embodiments, the nucleic acid is a siRNA or a nucleic acid encoding a siRNA. The siRNA may be conjugated to N-acetylgalactosamine.

In some embodiments, the method further comprises administering to the subject an antiviral agent. The antiviral agent may be an interferon or a nucleoside analogue, or a prodrug thereof. The antiviral agent may be pegylated interferon.

In some embodiments, the antiviral agent and the inhibitor are administered to the subject in separate compositions, or in combined compositions. The antiviral agent and the inhibitor may be administered to the subject sequentially or simultaneously.

In some embodiments, the subject is suffering from acute hepatitis B virus infection. In some embodiments, the subject is suffering from chronic hepatitis B virus infection.

The subject may be suffering from a hepatitis D virus infection.

Preferably, the subject is a human.

In a third aspect, the present disclosure provides a method of treating a hepatitis D virus infection in a subject the method comprising administering to the subject an inhibitor wherein the inhibitor inhibits or suppresses a regulator of liver lipid metabolism in the subject.

Preferably, the regulator is TM6SF2.

The inhibitor may be selected from the group consisting of an antibody or a fragment thereof, a nucleic acid and a small organic molecule. In certain embodiments, the inhibitor is a nucleic acid.

The nucleic acid may reduce accumulation of TM6SF2 mRNA in the subject.

In certain embodiments, the nucleic acid comprises at least 20 contiguous nucleotides which are substantially complementary to the nucleotide sequence set forth in SEQ ID NO. 3.

In some embodiments, the nucleic acid comprises at least 10 contiguous nucleotides differing by no more than 3 nucleotides from a nucleotide sequence set forth in any one of SEQ ID NOs. 1, 2 or 4. For example, the nucleic acid may comprise at least 10 contiguous nucleotides differing by no more than 3 nucleotides from a nucleotide sequence set forth in SEQ ID NO. 4. For example, the nucleic acid may comprise at least 20 contiguous nucleotides differing by no more than 3 nucleotides from a nucleotide sequence set forth in SEQ ID NO. 4.

In some embodiments, the nucleic acid comprises at least 10 contiguous nucleotides which are at least 80% identical to a nucleotide sequence set forth in any one of SEQ ID NOs 1, 2 or 4. For example, the nucleic acid may comprise at least 10 contiguous nucleotides which are at least 80% identical to a nucleotide sequence set forth in SEQ ID NO 4. For example, the nucleic acid may comprise at least 20 contiguous nucleotides which are at least 80% identical to a nucleotide sequence set forth in SEQ ID NO 4.

In certain embodiments, the nucleic acid comprises the sequence set forth in SEQ ID NO. 5 or SEQ ID NO. 6 and a sequence substantially complementary thereto.

The nucleic acid may be between 15 and 50 nucleotides in length.

In certain embodiments, the nucleic acid is a siRNA or a nucleic acid encoding a siRNA. The siRNA may be conjugated to N-acetylgalactosamine.

In certain embodiments, the method further comprises administering to the subject an antiviral agent. The antiviral agent may be an interferon or a nucleoside analogue. The antiviral agent may be pegylated interferon.

In some embodiments, the antiviral agent and the inhibitor are administered to the subject in separate compositions. The antiviral agent and the inhibitor may be administered to the subject sequentially or simultaneously.

Preferably, the subject is a human.

In a fourth aspect, the present disclosure provides a composition comprising an inhibitor and a pharmaceutically acceptable carrier wherein the inhibitor inhibits or suppresses a regulator of liver lipid metabolism. The regulator may regulate lipid synthesis and/or lipid secretion in the liver. The regulator may further regulate cholesterol synthesis and/or lipoprotein secretion in the liver.

Preferably, the regulator is TM6SF2.

The inhibitor may be selected from the group consisting of an antibody or a fragment thereof, a nucleic acid and a small organic molecule. In certain embodiments, the inhibitor is a nucleic acid.

The nucleic acid may comprise at least 20 contiguous nucleotides which are substantially complementary to the nucleotide sequence set forth in SEQ ID NO. 3.

In certain embodiments, the nucleic acid comprises at least 10 contiguous nucleotides differing by no more than 3 nucleotides from a nucleotide sequence set forth in any one of SEQ ID NOs. 1, 2 or 4. For example, the nucleic acid may comprise at least 10 contiguous nucleotides differing by no more than 3 nucleotides from a nucleotide sequence set forth in SEQ ID NO. 4. For example, the nucleic acid may comprise at least 20 contiguous nucleotides differing by no more than 3 nucleotides from a nucleotide sequence set forth in SEQ ID NO. 4.

In certain embodiments, the nucleic acid comprises at least 10 contiguous nucleotides which are at least 80% identical to a nucleotide sequence set forth in any one of SEQ ID NOs 1, 2 or 4. For example, the nucleic acid may comprise at least 10 contiguous nucleotides which are at least 80% identical to a nucleotide sequence set forth in SEQ ID NO 4. For example, the nucleic acid may comprise at least 20 contiguous nucleotides which are at least 80% identical to a nucleotide sequence set forth in SEQ ID NO 4.

The nucleic acid may comprise the sequence set forth in SEQ ID NO. 5 or SEQ ID NO. 6 and a sequence substantially complementary thereto.

In certain embodiments, the nucleic acid is between 15 and 50 nucleotides in length.

The nucleic acid may be a siRNA or a nucleic acid encoding a siRNA. The siRNA may be conjugated to N-acetylgalactosamine.

In some embodiments, the composition further comprises an antiviral agent. The antiviral agent is an interferon or a nucleoside analogue, or a prodrug thereof. The antiviral agent may be pegylated interferon.

The nucleic acid may reduce accumulation of TM6SF2 mRNA in the subject.

The nucleic acid may comprise at least 20 contiguous nucleotides which are substantially complementary to the nucleotide sequence set forth in SEQ ID NO. 3.

In certain embodiments, the nucleic acid comprises at least 10 contiguous nucleotides differing by no more than 3 nucleotides from a nucleotide sequence set forth in any one of SEQ ID NOs. 1, 2 or 4. For example, the nucleic acid may comprise at least 10 contiguous nucleotides differing by no more than 3 nucleotides from a nucleotide sequence set forth in SEQ ID NO. 4. For example, the nucleic acid may comprise at least 20 contiguous nucleotides differing by no more than 3 nucleotides from a nucleotide sequence set forth in SEQ ID NO. 4.

In certain embodiments, the nucleic acid comprises at least 10 contiguous nucleotides which are at least 80% identical to a nucleotide sequence set forth in any one of SEQ ID NOs 1, 2 or 4. For example, the nucleic acid may comprise at least 10 contiguous nucleotides which are at least 80% identical to a nucleotide sequence set forth in SEQ ID NO 4. For example, the nucleic acid may comprise at least 20 contiguous nucleotides which are at least 80% identical to a nucleotide sequence set forth in SEQ ID NO 4.

In certain embodiments, the nucleic acid comprises the sequence set forth in SEQ ID NO. 5 or SEQ ID NO. 6 and a sequence substantially complementary thereto.

The nucleic acid may be between 15 and 50 nucleotides in length.

The nucleic acid may be a siRNA or a nucleic acid encoding a siRNA.

In a seventh aspect, the present disclosure provides an isolated or recombinant nucleic acid for inhibiting TM6SF2.

The nucleic acid may reduce accumulation of TM6SF2 mRNA in the subject.

The nucleic acid may comprise at least 20 contiguous nucleotides which are substantially complementary to the nucleotide sequence set forth in SEQ ID NO. 3.

In certain embodiments, the nucleic acid comprises at least 10 contiguous nucleotides differing by no more than 3 nucleotides from a nucleotide sequence set forth in any one of SEQ ID NOs. 1, 2 or 4. For example, the nucleic acid may comprise at least 10 contiguous nucleotides differing by no more than 3 nucleotides from a nucleotide sequence set forth in SEQ ID NO. 4. For example, the nucleic acid may comprise at least 20 contiguous nucleotides differing by no more than 3 nucleotides from a nucleotide sequence set forth in SEQ ID NO. 4.

In certain embodiments, the nucleic acid comprises at least 10 contiguous nucleotides which are at least 80% identical to a nucleotide sequence set forth in any one of SEQ ID NOs 1, 2 or 4. For example, the nucleic acid may comprise at least 10 contiguous nucleotides which are at least 80% identical to a nucleotide sequence set forth in SEQ ID NO 4. For example, the nucleic acid may comprise at least 20 contiguous nucleotides which are at least 80% identical to a nucleotide sequence set forth in SEQ ID NO 4.

In certain embodiments, the nucleic acid comprises the sequence set forth in SEQ ID NO. 5 or SEQ ID NO. 6 and a sequence substantially complementary thereto.

The nucleic acid may be between 15 and 50 nucleotides in length.

The nucleic acid may be a siRNA or a nucleic acid encoding a siRNA.

The present invention relates to an inhibitor of transmembrane 6 superfamily member 2 (TM6SF2) for use in a method of treating a hepatitis B and/or hepatitis D virus infection in a subject, the method comprising administering to the subject said inhibitor, wherein the inhibitor is a nucleic acid or a small organic molecule.

Preferred embodiments of the invention are claimed in the present claim set.

### Brief description of drawings

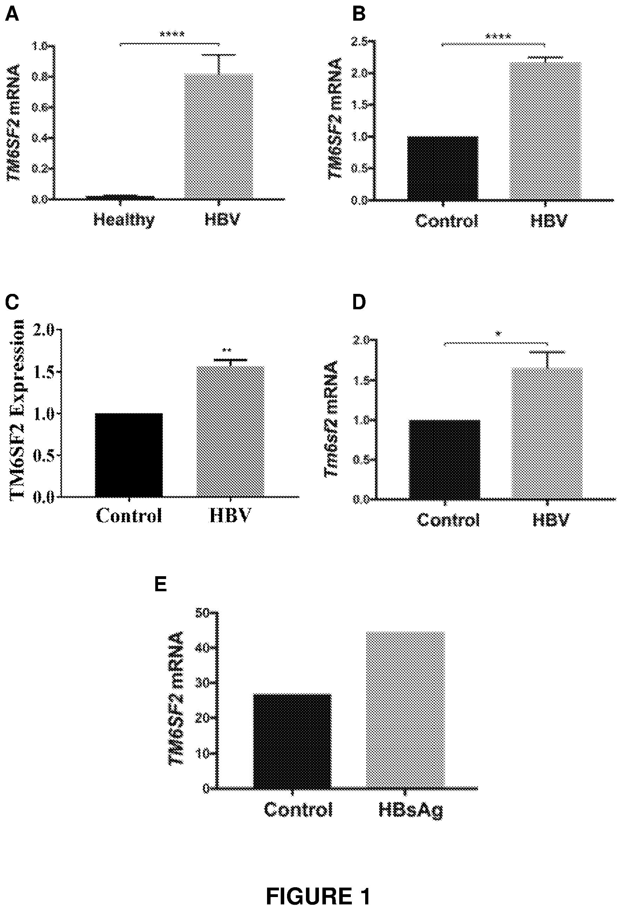

**Figure 1****:** A) TM6SF2 expression in liver biopsies from patients suffering from chronic hepatitis B; B) TM6SF2 expression in Huh7 cells transfected with HBV (genotype D); C) TM6SF2 expression in Huh7 cells transfected with HBV (genotype C); D) TM6SF2 expression in liver tissue from mice infected with HBV (genotype A); E) TM6S2 expression in HepG2 cells expressing HBsAg. (* p<0.05; **** p<0.0001).

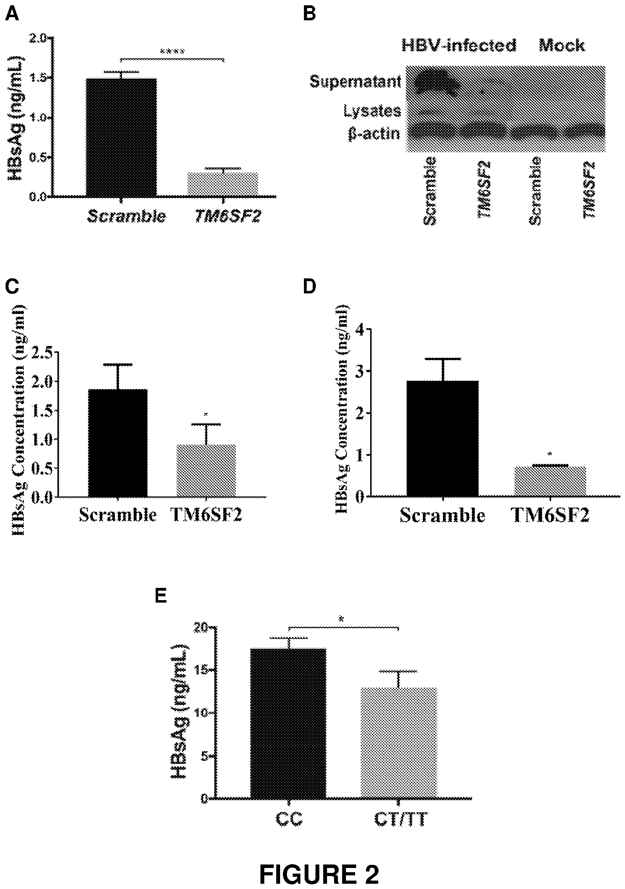

**Figure 2****:** A) HBsAg ELISA of supernatant from HBV (genotype D) infected Huh7 cells; B) HBsAg western blot of supernatant from HBV (genotype D) infected Huh7 cells and mock infected Huh7 cells, treated with TM6SF2 inhibitory siRNA or scramble control; C) HBsAg ELISA of supernatant from HBV (genotype C) infected Huh7 cells treated with TM6SF2 inhibitory siRNA or scramble control; D) HBsAg ELISA of supernatant from HBV (genotype D) infected HepaRG cells treated with TM6SF2 inhibitory siRNA or scramble control; E) HBsAg levels in serum samples from human subjects suffering from chronic hepatitis B. (* p<0.05; **** p<0.0001).

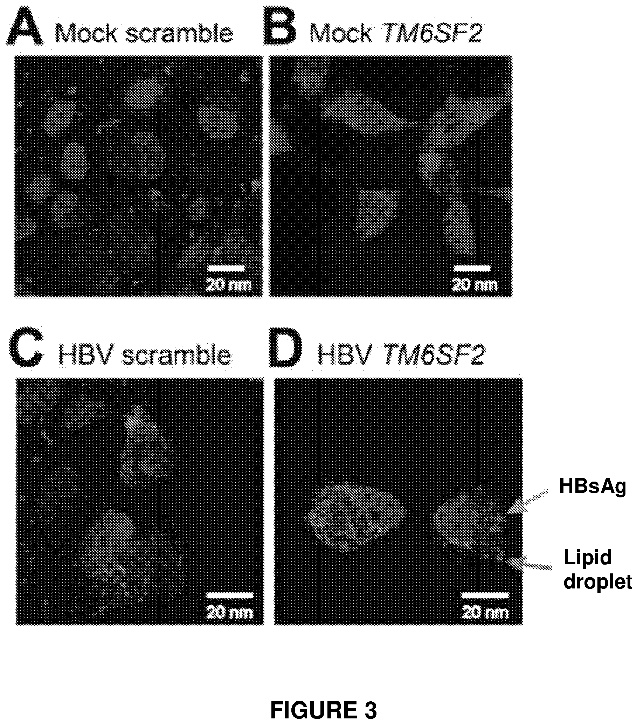

**Figure 3****:** Huh7 cells labelled with BODIPY 558/568 (red) for lipid droplets and for HBsAg (green).

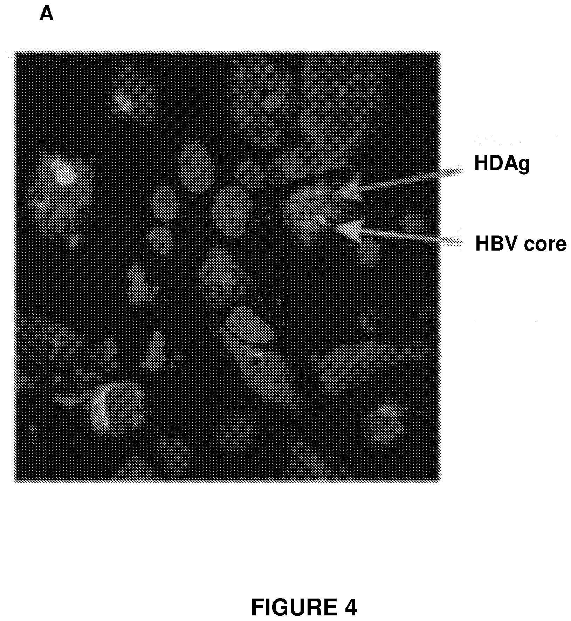

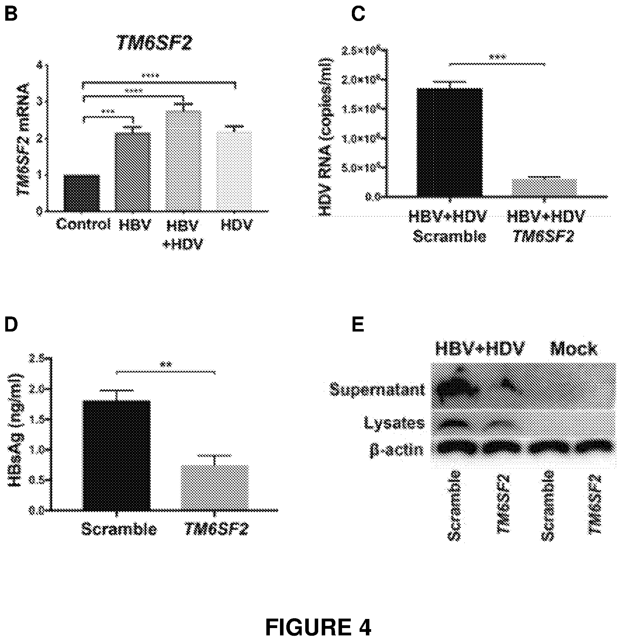

**Figure 4****:** A) Model of HDV/HBV co-infection in Huh7 cells (HDAg = hepatitis delta antigen); B) TM6SF2 expression in HDV/HBV co-infected Huh7 cells compared to cells infected with HBV or HDV alone; C) HDV RNA levels in Huh7 cells treated with TM6SF2 inhibitory siRNA or scramble control; D) HBsAg secretion from HDV/HBV co-infected Huh7 cells treated with TM6SF2 inhibitory siRNA or scramble control; E) Western blot of supernatant and cell lysate from HDV/HBV co-infected Huh7 cells treated with TM6SF2 inhibitory siRNA or scramble control. (** p<0.01; *** p<0.001; **** p<0.0001).

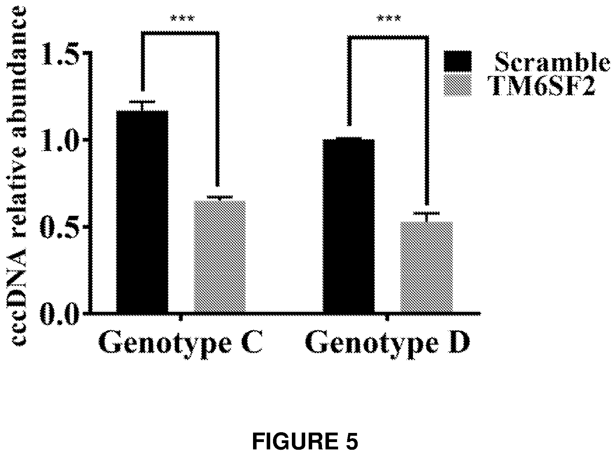

**Figure 5****:** HBV cccDNA levels in Huh7 cells treated with TM6SF2 inhibitor.

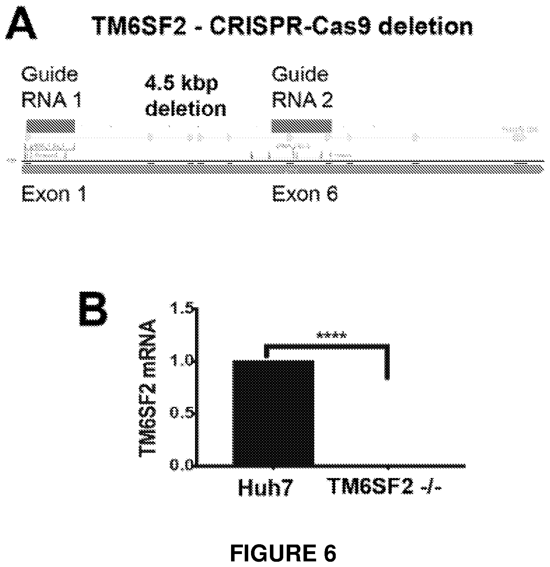

**Figure 6****:** A) Graphic representation of the position of guide RNAs relative to the TM6SF2 gene in Huh7 cells that were used to create a CRISPR-Cas9 4.5 kbp deletion. B) Measurement of TM6SF2 mRNA in Huh7 cells versus CRISPR-Cas9 modified TM6SF2 ^{-/-} Huh7 cells using qPCR showing (****p< 0.0001).

### Detailed description

### Definitions

In the context of this specification, the terms "a" and "an" are used herein to refer to one or to more than one (ie, to at least one) of the grammatical object of the article. By way of example, "an element" means one element or more than one element.

The term "about" is understood to refer to a range of +/- 10%, preferably +/- 5% or +/-1% or, more preferably, +/- 0.1%.

The terms "administration concurrently" or "administering concurrently" or "coadministering" and the like refer to the administration of a single composition containing two or more actives, or the administration of each active as separate compositions and/or delivered by separate routes either contemporaneously or simultaneously or sequentially within a short enough period of time that the effective result is equivalent to that obtained when all such actives are administered as a single composition. By "simultaneously" is meant that the active agents are administered at substantially the same time, and preferably together in the same formulation.

As used herein, the term "antibody" refers to any form of antibody that exhibits the desired biological activity. Thus, it is used in a broad sense and includes, but is not limited to, monoclonal antibodies (including full length monoclonal antibodies comprising two light chains and two heavy chains), polyclonal antibodies, multispecific antibodies (eg, bispecific antibodies), humanized antibodies, fully human antibodies, chimeric antibodies and camelized single domain antibodies. Single domain antibodies are composed of single VH or VL domains.

Naturally occurring antibody structural units typically comprise a tetramer. Each such tetramer typically comprises two pairs of polypeptide chains, each pair having one full-length "light" and one full-length "heavy" chain. The amino-terminal portion of each chain typically includes a variable region of about 100 to 110 or more amino acids that typically is responsible for antigen recognition. The carboxy-terminal portion of each chain typically defines a constant region that may be responsible for effector function. Human light chains are typically classified as kappa and lambda light chains. Heavy chains are typically classified as mu, delta, gamma, alpha or epsilon, and define the antibody's isotype as IgM, IgD, IgG, IgA and IgE, respectively. IgG has several subclasses, including, but not limited to, IgG1, IgG2, IgG3 and IgG4. IgM has subclasses including, but not limited to, IgM1 and IgM2. IgA is similarly subdivided into subclasses including, but not limited to, IgA1 and IgA2. Within full-length light and heavy chains, often, the variable and constant regions are joined by a "J" region of about 12 or more amino acids, with the heavy chain also including a "D" region of about 10 more amino acids. See, eg, Fundamental Immunology, Ch. 7 (Paul, W., ed., 2nd ed. Raven Press, N.Y. (1989)). The variable regions of each light/heavy chain pair typically comprise the antigen binding site.

As used herein, the term "antigen binding protein" refers to a protein that specifically binds to one or more target antigens. An antigen binding protein can include an antibody and binding fragments thereof. An "antigen binding fragment" or "antigen binding portion" used interchangeably in certain contexts herein with "binding fragment" or "fragment" is a portion of an antibody that lacks at least some of the amino acids present in a full-length heavy chain and/or light chain, but which is still capable of specifically binding to an antigen. An antigen binding fragment includes, but is not limited to, a single-chain variable fragment (scFv), a nanobody (eg, VH domain of camelid heavy chain antibodies; VHH fragment), a Fab fragment, a Fab' fragment, a F(ab')₂ fragment, a Fv fragment and a Fd fragment, and may be derived, for example, from a mammalian source, such as human, mouse, rat, rabbit or camelid. Antigen binding fragments may compete for binding to a target antigen with an intact antibody and the fragments may be produced by the modification of intact antibodies (eg, enzymatic or chemical cleavage) or synthesized de novo using recombinant DNA technologies or peptide synthesis.

An antigen binding protein may also include a protein comprising one or more antigen binding fragments incorporated into a single polypeptide chain or into multiple polypeptide chains. For example, antigen binding proteins may include, but are not limited to, a diabody (see, eg, EP 404,097; WO 93/11161; and Hollinger et al, Proc. Natl. Acad. Sci. USA, Vol. 90:6444- 6448, 1993), an intrabody, a domain antibody (single VL or VH domain or two or more VH domains joined by a peptide linker; see, eg, Ward et al, Nature, Vol. 341 :544-546, 1989), a maxibody (two scFvs fused to Fc region, see, eg, Fredericks et al, Protein Engineering, Design & Selection, Vol. 17:95-106, 2004 and Powers et al, J. Immunol. Meth. 2001. 251: 123-135), a triabody, a tetrabody, a minibody (scFv fused to CH3 domain; see, eg, Olafsen et al., Prot. Eng. Des. Sel. 2004. 17: 315-23), a peptibody (one or more peptides attached to an Fc region, see, eg, WO 00/24782), a linear antibody (a pair of tandem Fd segments (VH-CH₁-VH-CH₁) which, together with complementary light chain polypeptides, form a pair of antigen binding regions (see, eg, Zapata et al. Protein Eng. 1995. 8: 1057-1062), a small modular immunopharmaceutical (see, eg, U.S. Patent Publication No. 20030133939), and immunoglobulin fusion proteins (eg, IgG-scFv, IgG-Fab, 2scFv-IgG, 4scFv-lgG, VH-IgG, IgG-VH, and Fab-scFv-Fc; see, eg, Spiess et al, Mol. Immunol., Vol. 67(2 Pt A):95-106, 2015).

By "autologous" is meant something (eg, cells, tissues etc) derived from the same organism.

The terms "comprise", "comprises", "comprised" or "comprising", "including" or "having" and the like in the present specification and claims are used in an inclusive sense, ie, to specify the presence of the stated features but not preclude the presence of additional or further features.

The term "substantially complementary" when used to describe a first nucleotide sequence in relation to a second nucleotide sequence, refers to the ability of an oligonucleotide or polynucleotide comprising the first nucleotide sequence to hybridize to, and form a duplex structure with, an oligonucleotide or polynucleotide comprising the second nucleotide sequence. It will be understood that the sequence of a nucleic acid need not be 100% complementary to that of its target. Conditions under which hybridisation occurs may be stringent, such as 400 mM NaCl, 40 mM PIPES pH 6.4, 1 mM EDTA, 50°C or 70°C for 12-16 hours followed by washing. Other conditions, such as physiologically relevant conditions as may be encountered inside an organism, can also apply. Substantial complementarity allows the relevant function of the nucleic acid to proceed, eg, RNAi. The skilled person will be able to determine the set of conditions most appropriate for a test of complementarity of two sequences in accordance with the ultimate application of the hybridized nucleotides.

The term "host cell" includes an individual cell or cell culture which can be or has been a recipient of any recombinant vector(s) or isolated polynucleotide of the invention. Host cells include progeny of a single host cell, and the progeny may not necessarily be completely identical (genetically or morphologically) to the original parent cell due to natural, accidental, or deliberate mutation and/or change. A host cell includes cells transfected or infected *in vivo* or *in vitro* with a recombinant vector or a polynucleotide of the invention.

The term "identity" refers to a relationship between the sequences of two or more polypeptide molecules or two or more nucleic acid molecules, as determined by aligning and comparing the sequences. The percent identity between two sequences is a function of the number of identical positions shared by the sequences when the sequences are optimally aligned (ie, % homology = # of identical positions/total # of positions x 100), with optimal alignment determined taking into account the number of gaps, and the length of each gap, which need to be introduced for optimal alignment of the two sequences. The comparison of sequences and determination of percent identity between two sequences can be accomplished using a mathematical algorithm.

The percent identity between two nucleotide sequences can be determined using the GAP program in the GCG software package, using a NWSgapdna.CMP matrix and a gap weight of 40, 50, 60, 70, or 80 and a length weight of 1, 2, 3, 4, 5, or 6. The percent identity between two nucleotide or amino acid sequences can also be determined using the algorithm of E. Meyers and W. Miller (CABIOS, 4: 11-17 (1989)) which has been incorporated into the ALIGN program, using a PAM120 weight residue table, a gap length penalty of 12 and a gap penalty of 4. In addition, the percent identity between two amino acid sequences can be determined using the Needleman and Wunsch (J. Mol. Biol. (48):444-453 (1970)) algorithm which has been incorporated into the GAP program in the GCG software package, using either a Blossum 62 matrix or a PAM250 matrix, and a gap weight of 16, 14, 12, 10, 8, 6, or 4 and a length weight of 1, 2, 3, 4, 5, or 6.

The term "isolated" as used herein refers to material that is substantially or essentially free from components that normally accompany it in its native state. For example, an "isolated polynucleotide" as used herein refers to a polynucleotide which has been purified from the sequences which flank it in a naturally-occurring state, eg, a DNA fragment which has been removed from the sequences that are normally adjacent to the fragment. Alternatively, an "isolated peptide" or an "isolated polypeptide" and the like, as used herein, refer to *in vitro* isolation and/or purification of a peptide or polypeptide molecule from its natural cellular environment, and from association with other components of the cell, ie, it is not associated with *in vivo* substances.

The term "monoclonal antibody" (mAb) as used herein refers to an antibody obtained from a population of substantially homogeneous antibodies, ie, the individual antibodies comprising the population are identical except for possible naturally occurring mutations and/or post-translation modifications (eg, isomerizations, amidations) that may be present in minor amounts. Monoclonal antibodies are highly specific, being directed against a single antigenic site or determinant on the antigen (epitope), in contrast to polyclonal antibody preparations which typically include different antibodies directed against different epitopes. In addition to their specificity, monoclonal antibodies are typically synthesized by hybridoma culture, and are hence uncontaminated by other immunoglobulins. The term "monoclonal" indicates the character of the antibody as being obtained from a substantially homogeneous population of antibodies, and is not to be construed as requiring production of the antibody by any particular method.

The term "operably connected" or "operably linked" as used herein refers to the functional relationship between two or more nucleic acid segments such as a gene and a regulatory element including but not limited to a promoter, which then regulates the expression of the gene.

The term "pharmaceutically acceptable" as used herein refers to substances that do not cause substantial adverse allergic or immunological reactions when administered to a subject. A "pharmaceutically acceptable carrier" includes, but is not limited to, solvents, coatings, dispersion agents, wetting agents, isotonic and absorption delaying agents and disintegrants.

The term "polynucleotide variant" refers to polynucleotides displaying substantial sequence identity with a reference polynucleotide sequence or polynucleotides that hybridize with a reference sequence under stringent conditions. The term also encompasses polynucleotides that are distinguished from a reference polynucleotide by the addition, deletion or substitution of at least one nucleotide. Accordingly, the term "polynucleotide variant" includes polynucleotides in which one or more nucleotides have been added or deleted, or replaced with different nucleotides. In this regard, it is well understood in the art that certain alterations inclusive of mutations, additions, deletions and substitutions can be made to a reference polynucleotide whereby the altered polynucleotide retains the biological function or activity of the reference polynucleotide. The term "polynucleotide variant" also includes naturally occurring allelic variants. The terms "peptide variant" and "polypeptide variant" and the like refer to peptides and polypeptides that are distinguished from a reference peptide or polypeptide by the addition, deletion or substitution of at least one amino acid residue. In certain embodiments, a peptide or polypeptide variant is distinguished from a reference peptide or polypeptide by one or more substitutions, which may be conservative or non-conservative. In certain embodiments, the peptide or polypeptide variant comprises conservative substitutions and, in this regard, it is well understood in the art that some amino acids may be changed to others with broadly similar properties without changing the nature of the activity of the peptide or polypeptide. Peptide and polypeptide variants also encompass peptides and polypeptides in which one or more amino acids have been added or deleted, or replaced with different amino acid residues.

"Prevention" includes reduction of risk, incidence and/or severity of a condition or disorder. The terms "treatment" and "treat" include both prophylactic or preventive treatment (that prevent and/or slow the development of a targeted pathologic condition or disorder) and curative, therapeutic or disease-modifying treatment, including therapeutic measures that cure, slow down, lessen symptoms of, and/or halt progression of a pathologic condition or disorder; and treatment of patients at risk of contracting a disease or suspected to have contracted a disease, as well as patients who are ill or have been diagnosed as suffering from a disease or medical condition. The terms "treatment" and "treat" do not necessarily imply that a subject is treated until total recovery. The terms "treatment" and "treat" also refer to the maintenance and/or promotion of health in an individual not suffering from a disease but who may be susceptible to the development of an unhealthy condition. The terms "treatment" and "treat" are also intended to include the potentiation or otherwise enhancement of one or more primary prophylactic or therapeutic measures. As non-limiting examples, a treatment can be performed by a patient, a caregiver, a doctor, a nurse, or another healthcare professional.

The terms "RNA interference agent" and "RNAi agent" refer to an agent that contains RNA and which mediates the targeted degradation of an RNA transcript via an RNA-induced silencing complex (RISC) pathway. The RNAi agent directs the sequence-specific degradation of target RNA (eg, TM6SF2 mRNA) through a process known as RNA interference (RNAi).

Without wishing to be bound by theory it is believed that long double stranded RNA introduced into cells is broken down into small interfering RNA (siRNA) by a Type III endonuclease known as Dicer (Sharp et al. (2001) Genes Dev. 15:485). Dicer, a ribonuclease III-like enzyme, processes the dsRNA into approximately 19 to 23 base pair siRNAs with two-base 3' overhangs (Bernstein, et al., (2001) Nature 409:363), although that length and the extent of any 3' overhangs can vary. The siRNAs are then incorporated into an RNA-induced silencing complex (RISC) where one or more helicases unwind the siRNA duplex, enabling the complementary antisense strand to guide target recognition (Nykanen, et al., (2001) Cell 107:309). Upon binding to the appropriate target mRNA, the RISC can degrade the target and/or inhibit its translation to induce silencing (Elbashir, et al., (2001) Genes Dev. 15: 188).

The term "recombinant polynucleotide" as used herein refers to a polynucleotide formed *in vitro* by the manipulation of nucleic acid into a form not normally found in nature. For example, the recombinant polynucleotide may be in the form of an expression vector. Generally, such expression vectors include transcriptional and translational regulatory nucleic acid operably linked to the nucleotide sequence.

The term "recombinant polypeptide" as used herein refers to a polypeptide made using recombinant techniques, ie, through the expression of a recombinant polynucleotide.

The term "regulatory element" or "regulatory sequence" refers to a nucleic acid sequence which regulates expression of an operably linked coding sequence in a particular host cell. The regulatory sequences that are suitable for prokaryotic cells for example, include a promoter, and optionally a cis-acting sequence such as an operator sequence and a ribosome binding site. Control sequences that may be suitable for eukaryotic cells include promoters, polyadenylation signals, transcriptional enhancers, translational enhancers, leader or trailing sequences that modulate mRNA stability, as well as targeting sequences that target a product encoded by a transcribed polynucleotide to an intracellular compartment within a cell or to the extracellular environment.

A "therapeutically effective amount" is at least the minimum concentration or amount required to effect a measurable change and/or improvement of a particular disease or condition (eg, a HBV infection). A therapeutically effective amount herein may vary according to factors such as the disease state, age, sex, and weight of the patient, and the ability of the TM6SF2 inhibitor to elicit a desired response in the individual. A therapeutically effective amount is also one in which any toxic or detrimental effects of the TM6SF2 inhibitor are outweighed by the therapeutically beneficial effects.

The term "vector" refers to a nucleic acid molecule capable of transporting another nucleic acid to which it has been linked. One type of vector is a "plasmid", which refers to a circular double-stranded DNA loop into which additional nucleic acid segments can be ligated. Another type of vector is a viral vector; wherein additional nucleic acid segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. In some embodiments, vectors can be capable of directing the expression of nucleic acids to which they are operatively linked. Such vectors are referred to as "recombinant expression vectors", or more simply "expression vectors", which serve equivalent functions.

### Transmembrane 6 superfamily member 2

Transmembrane 6 superfamily member 2 (TM6SF2) is a protein involved in liver lipid metabolism. Its exact function is not known, but it appears to regulate cholesterol synthesis and secretion of lipoproteins (Li et al. (2017) Atherosclerosis 268, 170-176). In humans, TM6SF2 is expressed in the liver and intestine (Ehrardt et al. (2017) Human Mol Genet 26: 2719-2731).

The rs58542926 single nucleotide polymorphism (SNP), a C → T substitution, encodes an E167K amino acid substitution in the TM6SF2 protein, which reduces TM6SF2 levels in the liver. The polymorphism is associated with reduced blood lipid levels and reduced cardiovascular risk, but an increased risk of non-alcoholic fatty liver disease (Holmen et al. (2014) Nat Genet 46, 345-351; Sookoian et al. (2015) Hepatology 61, 515-525). The T allele has a frequency of 0.07, 0.04 and 0.02 in Europeans, Hispanics and Africans, respectively.

### Inhibitors of TM6SF2

The activity of the TM6SF2 protein may be inhibited in a number of different ways, and a TM6SF2 inhibitor may take a variety of forms. Although the inhibitor specifically inhibits TM6SF2 protein activity, it will be understood that it can do so by acting at the DNA, RNA or protein level. For example, the TM6SF2 inhibitor may target the TM6SF2 gene and reduce its expression or trigger polymorphisms which render the encoded TM6SF2 protein mal- or non-functional. Alternatively, the TM6SF2 inhibitor may inhibit TM6SF2 by targeting TM6SF2 mRNA for degradation. Still further, the TM6SF2 inhibitor may bind to TM6SF2 and inhibit its activity directly at a protein level.

In certain embodiments, the TM6SF2 inhibitor is a nucleic acid, such as DNA or RNA. The nucleic acid may target TM6SF2 mRNA and reduce TM6SF2 expression by RNAi. Alternatively, the nucleic acid may reduce TM6SF2 expression by guiding a transcriptionally-repressive CRISPR/Cas system to the TM6SF2 gene or to a DNA sequence adjacent the TM6SF2 gene. In still further embodiments, the nucleic acid may guide a nuclease-active CRISPR/Cas system to cause DNA modifications (eg, deletions) in or adjacent the TM6SF2 gene.

The nucleic acid of the present disclosure may comprise at least 10 contiguous nucleotides differing by no more than 3 nucleotides from a nucleotide sequence set forth in any one of SEQ ID NOs. 1, 2 or 4. In embodiments where the nucleic acid is RNA and SEQ ID NO. 1 and 2 refers to a deoxyribonucleotide (eg, T), it will be understood that the nucleic acid (ie, the RNA) may comprise its ribonucleotide equivalent (eg, U). In certain embodiments, the nucleic acid of the present disclosure comprises at least 10 contiguous nucleotides, such as at least 11 contiguous nucleotides, at least 12 contiguous nucleotides, at least 13 contiguous nucleotides, at least 14 contiguous nucleotides, at least 15 contiguous nucleotides, at least 16 contiguous nucleotides, at least 17 contiguous nucleotides, at least 18 contiguous nucleotides, at least 19 contiguous nucleotides, at least 20 contiguous nucleotides, at least 25 contiguous nucleotides, at least 30 contiguous nucleotides, at least 35 contiguous nucleotides, at least 40 contiguous nucleotides, or at least 45 contiguous nucleotides differing by no more than 3 nucleotides, such as less than 3 nucleotides or less than 2 nucleotides, from a nucleotide sequence set forth in any one of SEQ ID NOs. 1, 2 or 4. In embodiments where the inhibitor is a small RNA (eg, siRNA or miRNA), mismatches in the centre of the small RNA-target duplex may be more likely to reduce the efficacy of the small RNA compared to mismatches at the 3' end.

In certain embodiments, the nucleic acid of the present disclosure comprises at least 10 contiguous nucleotides which are at least 60% identical to a nucleotide sequence set forth in any one of SEQ ID NOs 1, 2 or 4. For example, the nucleic acid may comprise at least 10 contiguous nucleotides, such as at least 11 contiguous nucleotides, at least 12 contiguous nucleotides, at least 13 contiguous nucleotides, at least 14 contiguous nucleotides, at least 15 contiguous nucleotides, at least 16 contiguous nucleotides, at least 17 contiguous nucleotides, at least 18 contiguous nucleotides, at least 19 contiguous nucleotides, at least 20 contiguous nucleotides, at least 25 contiguous nucleotides, 30 contiguous nucleotides, at least 35 contiguous nucleotides, at least 40 contiguous nucleotides, or at least 45 contiguous nucleotides, which are at least 60% identical, such as at least 65% identical, at least 70% identical, at least 75% identical, at least 80% identical, at least 85% identical, at least 90% identical, at least 95% identical or 100% identical to a nucleotide sequence set forth in any one of SEQ ID NOs 1, 2 or 4.

In certain embodiments, the nucleic acid of the present disclosure comprises at least 10 contiguous nucleotides, such as at least 11 contiguous nucleotides, at least 12 contiguous nucleotides, at least 13 contiguous nucleotides, at least 14 contiguous nucleotides, at least 15 contiguous nucleotides, at least 16 contiguous nucleotides, at least 17 contiguous nucleotides, at least 18 contiguous nucleotides, at least 19 contiguous nucleotides, at least 20 contiguous nucleotides, at least 25 contiguous nucleotides, 30 contiguous nucleotides, at least 35 contiguous nucleotides, at least 40 contiguous nucleotides, or at least 45 contiguous nucleotides, which are substantially complementary to a nucleotide sequence set forth in any one of SEQ ID NOs. 1, 2 or 4. Preferably, the nucleic acid of the present disclosure is substantially complementary to the sequence set forth in SEQ ID NO. 3. In embodiments where the nucleic acid inhibitor is double stranded, it will be understood that only one strand is substantially complementary to the sequence set forth in SEQ ID NO. 3. The level of complementarity is preferably sufficient to enable the nucleic acid to hybridise to its target (eg, TM6SF2 DNA or RNA) and, for example, guide RNAi or genome editing. The nucleic acid may comprise a sense strand and an antisense strand, wherein the antisense strand is substantially complementary to the sequence set forth in SEQ ID NO. 3. In such embodiments, the antisense strand may comprise at least 10 contiguous nucleotides, at least 11 contiguous nucleotides, at least 12 contiguous nucleotides, at least 13 contiguous nucleotides, at least 14 contiguous nucleotides, at least 15 contiguous nucleotides, at least 16 contiguous nucleotides, at least 17 contiguous nucleotides, at least 18 contiguous nucleotides, at least 19 contiguous nucleotides or at least 20 contiguous nucleotides, which are at least 60% identical, such as at least 65% identical, at least 70% identical, at least 75% identical, at least 80% identical, at least 85% identical, at least 90% identical, at least 95% identical or 100% identical to SEQ ID NO. 4. The sense strand and the antisense strand form a double stranded region, but need not be perfectly complementary with each other. For example, the nucleic acid may comprise the sequence set forth in SEQ ID NO. 5 or SEQ ID NO. 6 and a sequence substantially complementary thereto.

In certain embodiments, the inhibitor of TM6SF2 is a siRNA or a nucleic acid encoding a siRNA. The siRNA may guide the degradation and/or translational repression of TM6SF2 mRNA by RNAi. The siRNA is preferably substantially complementary to TM6SF2 mRNA. For example, the siRNA may comprise the sequence set forth in SEQ ID NO. 5 or SEQ ID NO. 6 and a sequence substantially complementary thereto.

The nucleic acid of the present disclosure may be between 10 and 50 nucleotides in length. It will be understood that the nucleic acid may be double stranded and formed from separate complementary strands, in which case, the length of the nucleic acid is made with reference to one strand. For example, in embodiments where the nucleic acid is between 10 and 50 nucleotides in length, each strand will be between 10 and 50 nucleotides in length. In certain embodiments, the nucleic acid of the present disclosure is between 10 and 1,000 nucleotides in length. For example, the nucleic acid may be between 10 and 900 nucleotides, between 10 and 800 nucleotides, between 10 and 700 nucleotides, between 10 and 600 nucleotides, between 10 and 500 nucleotides, between 10 and 400 nucleotides, between 10 and 300 nucleotides, between 10 and 200 nucleotides, between 10 and 100 nucleotides, between 10 and 90 nucleotides, between 10 and 80 nucleotides, between 10 and 70 nucleotides, between 10 and 60 nucleotides, between 10 and 50 nucleotides, between 10 and 40 nucleotides, or between 10 and 30 nucleotides in length. The nucleic acid may be, or each stand of a double stranded nucleic acid may independently be, for example, 15 nucleotides, 16 nucleotides, 17 nucleotides, 18 nucleotides, 19 nucleotides, 20 nucleotides, 21 nucleotides, 22 nucleotides, 23 nucleotides, 24 nucleotides, 25 nucleotides, 26 nucleotides, 27 nucleotides, 28 nucleotides, 29 nucleotides or 30 nucleotides in length.

In embodiments where the nucleic acid is dsRNA, the dsRNA may comprise one or two overhang regions. The overhang regions may be 1 to 6 nucleotides in length, such as 2 to 6 nucleotides, 1 to 5 nucleotides, 2 to 5 nucleotides, 1 to 4 nucleotides, 2 to 4 nucleotides, 1 to 3 nucleotides, 2 or 3 nucleotides or 1 or 2 nucleotides in length. The overhangs can be the result of one strand being longer than the other, or the result of two strands of the same length being staggered. In one embodiment, the nucleotides in the overhang region of the RNA are each independently a modified or unmodified nucleotide including, but no limited to 2'-sugar modified, such as, 2-F,2'-O-methyl, thymidine (T), deoxy-thymine (dT), 2'-O-methoxyethyl-5-methyluridine (Teo), 2'-O-methoxyethyladenosine (Aeo), 2'-O-methoxyethyl-5-methylcytidine (m5Ceo), and any combinations thereof. For example, dTdT can be an overhang sequence for either end on either strand. In some embodiments, the overhang region(s) contains two nucleotides having a phosphorothioate between the two nucleotides, where the two nucleotides can be the same or different. In some embodiments, the RNA contains only a single overhang, which can strengthen the interference activity of the RNA, without affecting its overall stability.

In certain embodiments, the nucleic acid comprises modifications, for example, end modifications, eg, 5'-end modifications (phosphorylation, conjugation, inverted linkages) or 3'-end modifications (conjugation, DNA nucleotides, inverted linkages, etc.); base modifications, eg, replacement with stabilizing bases, destabilizing bases, or bases that base pair with an expanded repertoire of partners, removal of bases (abasic nucleotides), or conjugated bases; sugar modifications (eg, at the 2'-position or 4'-postion) or replacement of the sugar; and/or backbone modifications, including modification or replacement of the phosphodiester linkages. Modified RNA backbones that do not include a phosphorus atom therein have backbones that are formed by short chain alkyl or cycloalkyl internucleoside linkages, mixed heteroatoms and alkyl or cycloalkyl internucleoside linkages, or one or more short chain heteroatomic or heterocyclic internucleoside linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N**,** O, S and CH₂ component parts.

In peptide nucleic acid (PNA) compounds, the sugar backbone of a RNA is replaced with an amide containing backbone, in particular an aminoethylglycine backbone. The nucleobases are retained and are bound directly or indirectly to aza nitrogen atoms of the amide portion of the backbone. Modified RNAs can also contain one or more substituted sugar moieties. The RNA may include one of the following at the 2'-position: OH; F; O-, S-, or N-alkyl; O-, S-, or N-alkenyl; O-, S- or N-alkynyl; or O-alkyl-O-alkyl, wherein the alkyl, alkenyl and alkynyl can be substituted or unsubstituted C₁ to C₁₀ alkyl or C₂ to C₁₀ alkenyl and alkynyl. A RNA can also comprise nucleobase (base) modifications. Modified nucleobases include other synthetic and natural nucleobases such as deoxy-thymine (dT), 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine, 6-azo uracil, cytosine and thymine, 5-uracil (pseudouracil), 4-thiouracil, 8-halo, 8-amino, 8-thiol, 8-thioalkyl, 8-hydroxyl anal other 8-substituted adenines and guanines, 5-halo, particularly 5-bromo, 5-trifluoromethyl and other 5-substituted uracils and cytosines, 7-methylguanine and 7-methyladenine, 8-azaguanine and 8-azaadenine, 7-deazaguanine and 7-daazaadenine and 3-deasaguanine and 3-deazaadenine.

The RNA may also be modified to include one or more locked nucleic acids (LNA). A locked nucleic acid is a nucleotide having a modified ribose moiety in which the ribose moiety comprises an extra bridge connecting the 2' and 4' carbons. This structure effectively "locks" the ribose in the 3'-endo structural conformation. The addition of locked nucleic acids to siRNAs has been shown to increase siRNA stability in serum, and to reduce off-target effects (Elmen, et al., (2005) Nucleic Acids Research 33(1):439-447). Potentially stabilizing modifications to the ends of RNA can include N-(acetylaminocaproyl)-4-hydroxyprolinol (Hyp-C6-NHAc), N-(caproyl-4-hydroxyprolinol (Hyp-C6), N-(acetyl-4-hydroxyprolinol (Hyp-NHAc), thymidine-2'-O-deoxythymidine (ether), N-(aminocaproyl)-4-hydroxyprolinol (Hyp-C6-amino), inverted base dT(idT) and others. In preferred embodiments, the nucleic acid of the present disclosure is conjugated to N-acetylgalactosamine (GalNAc) or a GalNAc derivative. The GalNAc may be attached to the nucleic acid via a monovalent linker, a bivalent linker or a trivalent linker.

In certain embodiments, the inhibitor of TM6SF2 is a nucleic acid comprising at least 15 contiguous nucleotides which are substantially complementary to a sequence within TM6SF2 mRNA (SEQ ID NO. 3), wherein the nucleic acid is between 20 and 50 nucleotides in length. The mRNA sequence of a common TM6SF2 allele is set forth in SEQ ID NO. 3. However, those skilled in the art will understand that other TM6SF2 alleles exist within the human population and that the present disclosure is not limited to a particular allele. In certain embodiments, the inhibitor of TM6SF2 may be an RNA comprising at least 20 contiguous nucleotides which are substantially complementary to the nucleotide sequence set forth in SEQ ID NO. 3, wherein the RNA is between 20 and 50 nucleotides in length. For example, the inhibitor of TM6SF2 may be an siRNA or a vector encoding an siRNA comprising at least 20 contiguous nucleotides which are substantially complementary to the nucleotide sequence set forth in SEQ ID NO. 3, wherein the siRNA is between 20 and 30 nucleotides in length. For example, the inhibitor of TM6SF2 may be an siRNA or a vector encoding an siRNA comprising at least 20 contiguous nucleotides which are substantially complementary to the nucleotide sequence set forth in SEQ ID NO. 3, wherein the siRNA is between 20 and 30 nucleotides in length, and wherein the siRNA or the vector is conjugated to N-acetylgalactosamine. In some embodiments, the inhibitor of TM6SF2 is a nucleic acid comprising at least 10 contiguous nucleotides which are at least 80% identical to a nucleotide sequence set forth in SEQ ID NO 4. For example, the inhibitor of TM6SF2 may be a nucleic acid comprising at least 20 contiguous nucleotides which are at least 80% identical to a nucleotide sequence set forth in SEQ ID NO 4, wherein the nucleic acid is between 20 and 50 nucleotides in length. For example, the inhibitor of TM6SF2 may be an siRNA or a vector encoding an siRNA comprising at least 20 contiguous nucleotides which are at least 80% identical to a nucleotide sequence set forth in SEQ ID NO 4, wherein the siRNA is between 20 and 30 nucleotides in length, and wherein the siRNA or the vector is conjugated to N-acetylgalactosamine.

The nucleic acid may be modified or unmodified, RNA or DNA, isolated, synthesised or recombinant. In embodiments where the nucleic acid is RNA, that RNA may be administered to a subject either directly or as DNA molecule which is transcribed to produce the RNA. The RNA may be single stranded (ss) or double stranded (ds). The RNA may be substantially complementary to TM6SF2 mRNA and thereby target the TM6SF2 mRNA, blocking its translation and/or triggering its degradation by RNAi. Administration of the inhibitory nucleic acid to a subject may therefore reduce accumulation of TM6SF2 RNA in the subject. Those skilled in the art will be aware of various techniques that may be used to measure RNA accumulation, for example, qPCR, Northern blotting, microarray, nucleic acid sequencing etc. In some embodiments, the nucleic acid of the present disclosure reduces accumulation of TM6SF2 RNA in the subject by at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or more. The reduction in TM6SF2 RNA may be measured, for example, by comparing two populations of cells; one control (untreated) population and one population which is treated with the nucleic acid, such that the level of mRNA reduction is measured as: [(mRNA in control cells) - (mRNA in treated cells)] ÷ (mRNA in control cells) x 100%.

The nucleic acid of the present disclosure may be a small interfering RNA (siRNA) or a nucleic acid encoding a siRNA. RNAi constructs may be produced by chemical synthetic methods or by recombinant nucleic acid techniques. Endogenous RNA polymerase of a treated cell may mediate transcription *in vivo,* or cloned RNA polymerase may be used for *in vitro* transcription. The RNAi constructs may include modifications to either the phosphate-sugar backbone or the nucleoside, for example, to reduce susceptibility to cellular nucleases, improve bioavailability, improve formulation characteristics, or to change other pharmacokinetic properties. The RNAi construct may encode a long dsRNA. In certain embodiments, the RNAi construct encodes a dsRNA that is at least 20, 25, 30, 50, 100, 200, 300 or 400 nucleotides in length. Long dsRNAs produced by such constructs may be digested intracellularly to produce small RNAs in the cell. In certain embodiments, the RNAi construct may encode a hairpin RNA.

Other nucleic acids that may be used to reduce TM6SF2 expression include guide RNAs, which may be introduced to a cell either directly or by way of a DNA construct encoding the guide RNA, along with a DNA endonuclease such as Cas9 or Cpf1. Alternatively, the guide RNA may be used to guide a protein which represses the transcription or translation of TM6SF2 when bound to the TM6SF2 gene or transcript. In that regard, the guide RNA may be engineered to include a protein binding sequence such as an aptamer, enabling it to bind one or more proteins which repress the transcription of TM6SF2 as well as to the DNA endonuclease, which may be rendered enzymatically inactive (Konermann et al. Nature (2015) 517: 583-588). The guide RNA may include a guide sequence (often referred to as a spacer) which is substantially complementary to the TM6SF2 gene or to genomic DNA flanking the TM6SF2 gene.

Zinc finger nucleases (ZFNs) may also be employed to reduce the expression of TM6SF2. Zinc finger nucleases are modular proteins comprised of an engineered zinc finger DNA binding domain linked to the catalytic domain of the type II endonuclease Fokl. Because Fokl functions as a dimer, a pair of ZFNs can be engineered to bind to cognate target "half-site" sequences on opposite DNA strands and with precise spacing between them to enable the catalytically active Fokl dimer to form. Upon dimerization of the Fokl domain, which itself has no sequence specificity per se, a DNA double-strand break is generated between the ZFN half-sites as the initiating step in genome editing. Transcription Activator-Like Effector Nucleases (TALENs) represent another format of modular nucleases whereby, as with ZFNs, an engineered DNA binding domain is linked to the Fokl nuclease domain, and a pair of TALENs operate in tandem to achieve targeted DNA cleavage.

The delivery of nucleic acid of the present disclosure to a cell, eg, a cell within a subject, such as a human subject can be achieved in a number of different ways. *In vivo* delivery may be performed directly by administering a composition comprising the nucleic acid to a subject. Alternatively, in embodiments where the inhibitor of TM6SF2 is RNA, *in vivo* delivery may be performed indirectly by administering one or more DNA vectors that encode and direct the expression of the RNA.

Factors to consider for *in vivo* delivery include biological stability of the delivered molecule, prevention of non-specific effects and accumulation of the delivered molecule in the target tissue. The non-specific effects of the nucleic acid can be minimized by local administration, for example, by direct injection or implantation into a tissue. Modification of the nucleic acid or the pharmaceutical carrier can also permit tissue-specific targeting reduced off-target effects. Nucleic acid molecules can be modified by chemical conjugation to lipophilic groups such as cholesterol to enhance cellular uptake and to prevent degradation (see, eg, Soutschek, et al. (2004) Nature 432:173-178).

In an alternative embodiment, the nucleic acid can be delivered using drug delivery systems such as a nanoparticle, a dendrimer, a polymer, liposomes, or a cationic delivery system. Cationic delivery systems facilitate binding of a nucleic acid molecule and also enhance interactions at the negatively charged cell membrane to permit efficient uptake of the nucleic acid by the cell. Cationic lipids, dendrimers, or polymers can either be bound to a nucleic acid, or induced to form a vesicle or micelle (see eg, Kim, et al. (2008) J Controlled Release 129(2): 107-116) that encases a nucleic acid. The formation of vesicles or micelles further prevents degradation of the nucleic acid when administered systemically. Methods for making and administering cationic nucleic acid complexes can readily be performed by those skilled in the art (see eg, Sorensen, et al. (2003) J. Mal. Biol. 327:761-766; Verma, et al. (2003) Clin. Cancer Res. 9:1291-1300; Arnold, et al. (2007) J. Hypertens. 25: 197-205). Some non-limiting examples of drug delivery systems useful for systemic delivery of RNA include DOTAP (Sorensen, et al. (2003) J. Mal. Biol. 327:761-766; Verma, et al. (2003) Clin. Cancer Res. 9:1291-1300), oligofectamine, solid nucleic acid lipid particles (Zimmermann, et al. (2006) Nature 441: 111-114), cardiolipin (Chien, et al. (2005) Cancer Gene Ther. 12:321-328; Pal, et al. (2005) Int J. Oneal. 26: 1087-1091), polyethyleneimine (Aigner, (2006) J. Biomed. Biotechnol. 71659), Arg-Gly-Asp (RGD) peptides (Liu, (2006) Mal. Pharm. 3:472-487), and polyamidoamines (Tomalia, et al. (2007) Biochem. Soc. Trans. 35:61-67). In some embodiments, the nucleic acid forms a complex with cyclodextrin for systemic administration. In some embodiments, the nucleic acid of the present disclosure is formulated with a lipid nanoparticle composition comprising a cationic lipid/Cholesterol/PEG-C-DMA/DSPC (eg, in a 40/48/2/10 ratio), a cationic lipid/Cholesterol/PEG-DMG/DSPC (eg, in a 40/48/2/10 ratio), or a cationic lipid/Cholesterol/PEG-DMG (eg, in a 60/38/2 ratio). In some embodiments, the cationic lipid is Octyl CL in DMA, DL in DMA, L-278, DLinKC2DMA or MC3. In certain embodiments, the nucleic acid is conjugated to, or complexed with, another compound, eg, to facilitate delivery of the nucleic acid (eg, CDM-LBA, CDM-Pip-LBA, CDM-PEG, CDM-NAG etc.). In certain embodiments, polyethylene glycol (PEG) is covalently attached to the nucleic acid. In further embodiments, the nucleic acid is formulated or complexed with polyethyleneimine or a derivative thereof, such as polyethyleneimine-polyethyleneglycol-N-acetylgalactosamine (PEI-PEG-GAL) or polyethyleneimine-polyethyleneglycol-tri-N-acetylgalactosamine (PEI-PEG-triGAL) derivatives.

RNA targeting TM6SF2 can also be expressed from a DNA vector. Expression can be transient (in the order of hours to weeks) or sustained (weeks to months or longer), depending upon the specific construct used and the target tissue or cell type. Transgenes expressing the RNA can be introduced as a linear construct, a circular plasmid, or a viral vector, which can be an integrating or non-integrating vector. The transgene can also be constructed to permit inheritance as an extrachromosomal plasmid (Gassmann, et al., Proc. Natl. Acad. Sci. USA (1995) 92:1292). The individual strand or strands of an RNA can be transcribed from a promoter on an expression vector. Where two separate strands are to be expressed to generate, for example, a dsRNA, two separate expression vectors can be co-introduced (eg, by transfection or infection) into a target cell. Alternatively each individual strand of a dsRNA can be transcribed by promoters, both of which are located on the same expression plasmid. In one embodiment, a dsRNA is expressed as inverted repeat polynucleotides joined by a linker polynucleotide sequence such that the dsRNA has a stem and loop structure.

RNA expression vectors are generally DNA plasmids or viral vectors. Expression vectors compatible with eukaryotic cells, preferably those compatible with vertebrate cells, can be used to produce recombinant constructs for the expression of an RNA as described herein. Classes of viral systems that are used in gene therapy can be categorized into two groups according to whether their genomes integrate into host cellular chromatin (oncoretroviruses and lentiviruses) or persist in the cell nucleus predominantly as extrachromosomal episomes (adeno-associated virus, adenoviruses and herpesviruses). Adenoviruses are medium sized double-stranded, non-enveloped DNA viruses with linear genomes that are between 26-48 Kbp. As described herein, the viral vector is from the Parvoviridae family. The Parvoviridae is a family of small single-stranded, non-enveloped DNA viruses with genomes approximately 5000 nucleotides long. As described herein, the viral vector is from the family Retroviridae. Retroviruses comprise single-stranded RNA animal viruses that are characterized by two unique features. First, the genome of a retrovirus is diploid. Second, this RNA is transcribed by the virion-associated enzyme reverse transcriptase into double-stranded DNA. This dsDNA or provirus can then integrate into the host genome and be passed from parent cell to progeny cells as a stably-integrated component of the host genome. Lentivirus vectors are often pseudotyped with vesicular steatites virus glycoprotein (VSV-G), and have been derived from the human immunodeficiency virus (HIV); visan-maedi, which causes encephalitis (visna) or pneumonia in sheep; equine infectious anemia virus (EIAV), which causes autoimmune hemolytic anemia and encephalopathy in horses; feline immunodeficiency virus (FIV), which causes immune deficiency in cats; bovine immunodeficiency virus (BIV) which causes lymphadenopathy and lymphocytosis in cattle; and simian immunodeficiency virus (SIV), which causes immune deficiency and encephalopathy in non-human primates. As described herein, the viral construct comprises an inactivated or self-inactivating 3' LTR from a lentivirus. The 3' LTR may be made self-inactivating by any method known in the art.

In other embodiments, the TM6SF2 inhibitor of the present disclosure is a small organic molecule. A small organic molecule generally has a low molecular weight such as less than 5000 Daltons, less than 4000 Daltons, less than 3000 Daltons, less than 2000 Daltons or less than 1000 Daltons.

Inhibitors may be identified by screening a combinatorial library containing a large number of potentially effective molecules. Such combinatorial chemical libraries can be screened in one or more assays to identify those library members (particular chemical species or subclasses) that display a desired characteristic activity such as TM6SF2 binding. The molecules thus identified can serve as conventional "lead compounds" or can themselves be used to inhibit TM6SF2.

Preparation and screening of combinatorial chemical libraries is well known to those of skill in the art. Such combinatorial chemical libraries include, but are not limited to, peptide libraries (see, eg, U.S. Pat. No. 5,010,175; Furka, Int. J. Pept. Prot. Res. 1991. 37:487-493 and Houghton et al. Nature, 1991. 354:84-88) and carbohydrate libraries (see, eg, Liang et al. Science, 1996. 274:1520-1522 and U.S. Pat. No. 5,593,853). Other chemistries for generating diverse chemical libraries can also be used, for example, peptoids (see, eg, WO 91/19735), encoded peptides (see, eg, WO 93/20242), random bio-oligomers (see, eg, WO 92/00091), benzodiazepines (see, eg, U.S. Pat. No. 5,288,514), diversomers such as hydantoins, benzodiazepines and dipeptides (see, eg, Hobbs et al., Proc. Nat. Acad. Sci. 1993. USA 90: 6909-6913), vinylogous polypeptides (see, eg, Hagihara et al., J. Amer. Chem. Soc. 1992. 114:6568), nonpeptidal peptidomimetics with β-D-glucose scaffolding (see, eg, Hirschmann et al., J. Amer. Chem. Soc. 1992. 114:9217-9218), analogous organic syntheses of small compound libraries (see, eg, Chen et al., J. Amer. Chem. Soc. 1994. 116:2661), oligocarbamates (see, eg, Cho et al., Science 1993. 261:1303), and/or peptidyl phosphonates (see, eg, Campbell et al., J. Org. Chem. 1994. 59:658), nucleic acid libraries (see, eg, Ausubel et al., eds., Current Protocols in Molecular Biology (1994); Sambrook and Russell, Molecular Cloning, A Laboratory Manual, 3rd ed. 2001), peptide nucleic acid libraries (see, eg, U.S. Pat. No. 5,539,083), antibody libraries (see, eg, Vaughn et al., Nat. Biotech. 1996. 14(3):309-314 and PCT/US96/10287), virtual ligand screening (see, eg, Klebe, Drug Discov. Today. 2006. 11(13-14): 580-594; Tran et al. Int. J. Med. Sci. 2015. 12(2): 163-176; Menchon et al. Sci. Rep. 2016. 6:22878) and small organic molecule libraries (see, eg, Baum C&EN, January 18, (1993); U.S. Pat. No. 5,569,588; U.S. Pat. No. 5,549,974; U.S. Pat. No. 5,525,735; U.S. Pat. No. 5,519,134; U.S. Pat. No. 5,506,337; U.S. Pat. No. 5,288,514).

Other suitable binding molecules may include peptide analogs. Peptide analogs are commonly used in the pharmaceutical industry as non-peptide drugs with properties analogous to those of the reference or template peptide. These types of non-peptide compounds are sometimes referred to as "peptide mimetics" or "peptidomimetics", and they are often developed with the aid of computerized molecular modelling. Generally, peptidomimetics are structurally similar to a paradigm polypeptide (eg, a polypeptide that has a biochemical property such as a binding capability), such as a human antibody, but have one or more peptide linkages optionally replaced by a linkage such as: -CH₂NH-, -CH₂S-, - CH₂-CH₂-, -CH=CH-(cis and trans), -COCH₂-, -CH(OH)CH₂--, or -CH₂SO-, by methods known in the art. Systematic substitution of one or more amino acids of a consensus sequence with a D-amino acid of the same type (eg, D-lysine in place of L-lysine) may be used to generate more stable peptides. In addition, constrained peptides comprising a consensus sequence or a substantially identical consensus sequence variation may be generated by methods known in the art (see, eg, Rizo and Gierasch, Ann. Rev. Biochem., 1992. 61:387), for example, by adding internal cysteine residues capable of forming intramolecular disulfide bridges which cyclize the peptide.

Antibodies or fragments thereof may also be developed which bind to TM6SF2 and thereby inhibit its activity. Those skilled in the art will be aware of various methods that may be used to generate antibodies and antigen binding proteins. For example, antibodies may be produced by immunising a non-human animal, eg, a rodent, with TM6SF2 or a fragment thereof. In certain embodiments, the anti-TM6SF2 antibody is a monoclonal antibody. Monoclonal antibodies can be produced using a variety of techniques known in the art including by using hybridoma technologies, recombinant DNA technologies, and phage display technologies, or a combination thereof. Other techniques for producing human monoclonal antibodies include the trioma technique, the human B-cell hybridoma technique and the EBV-hybridoma technique. Phage display is described in U.S. Patent No. 5,223,409; Smith Science. 1985. 228:1315-1317, Clackson et al. Nature. 1991. 352: 624-628 and Marks et al. J. Mol. Biol. 1991. 222: 581-597.

Those skilled in the art will be aware of several techniques for producing monoclonal antibodies using hybridoma technology. By way of non-limiting example, splenocytes and/or lymph node cells from immunized mice may be isolated and fused to an appropriate immortalized cell line, such as a mouse myeloma cell line. The resulting hybridomas can be screened for the production of antigen-specific antibodies. For example, single cell suspensions of splenic lymphocytes from immunized mice can be fused to Sp2/0 nonsecreting mouse myeloma cells (ATCC CRL 1581) with 50% PEG. Cells may be plated at approximately 2 x 10⁵ in a flat bottom microtiter plate, followed by a two week incubation in selective medium containing 10% fetal Clone Serum, 18% "653" conditioned media, 5% origen (IGEN), 4 mM L-glutamine, 1 mM sodium pyruvate, 5mM HEPES, 0.055 mM 2-mercaptoethanol, 50 units/ml penicillin, 50 mg/ml streptomycin, 50 mg/ml gentamycin and 1X HAT (Sigma). After approximately two weeks, cells can be cultured in medium in which the HAT is replaced with HT. Individual wells can then be screened by ELISA for human monoclonal IgM and IgG antibodies. Once extensive hybridoma growth occurs, the medium may be observed after about 10-14 days. The antibody secreting hybridomas can be replated, screened again, and if still positive for human IgG, the monoclonal antibodies can be subcloned at least twice by limiting dilution. The stable subclones can then be cultured *in vitro* to generate small amounts of antibody in tissue culture medium for characterization. To purify monoclonal antibodies, selected hybridomas can be grown in two litre spinner-flasks for monoclonal antibody purification. Supernatants can be filtered and concentrated before affinity chromatography with protein A-Sepharose. The buffer solution can be exchanged into PBS, and the concentration can be determined by OD280 using 1.43 extinction coefficient.

Hybridomas may be screened using standard methods, such as ELISA and surface plasmon resonance (BIACORE), to identify hybridomas that produce an antibody that specifically binds to a particular target such as TM6SF2. Surface plasmon resonance may also be used to increase the efficiency of phage antibodies which bind to an epitope of TM6SF2.

Chimeric or humanized antibodies can be prepared based on the sequence of a murine monoclonal antibody. DNA encoding the heavy and light chain immunoglobulins can be obtained from the murine hybridoma of interest and engineered to contain non-murine (eg, human) immunoglobulin sequences using standard molecular biology techniques. For example, to create a chimeric antibody, the murine variable regions can be linked to human constant regions using methods known in the art. To create a humanized antibody, the murine CDR regions can be inserted into a human framework using methods known in the art (see, eg, U.S. Patent Nos. 5,225,539; 5,530,101; 5,585,089; 5,693,762 and 6,180,370).

For antibodies expressed by hybridomas, cDNAs encoding the light and heavy chains of the antibody may be obtained by standard PCR amplification or cDNA cloning techniques. For antibodies obtained from an immunoglobulin gene library (eg, using phage display techniques), nucleic acid encoding the antibody may be recovered from the library. Once DNA fragments encoding VH and VL segments are obtained, they may be manipulated by standard recombinant DNA techniques, for example, to convert the variable region genes to full-length antibody chain genes, to Fab fragment genes or to a scFv gene. In these manipulations, a VL- or VH-encoding DNA fragment may be linked to another DNA fragment encoding another protein, such as an antibody constant region or a flexible linker.

Humanized antibodies or fragments thereof may be generated by replacing sequences of the Fv variable domain that are not directly involved in antigen binding with equivalent sequences from human Fv variable domains. The humanized antibodies may be expressed from nucleic acid sequences that encode all or part of immunoglobulin Fv variable domains from at least one of a heavy or light chain. Such nucleic acids may be obtained from a hybridoma producing an antibody against a target antigen such as TM6SF2, as well as from other sources. The recombinant DNA encoding the humanized antibody molecule can then be cloned into an appropriate expression vector.

Humanized antibodies may also be produced using transgenic animals (eg, mice) which are engineered to express human heavy and light chain genes, but are incapable of expressing endogenous mouse immunoglobulin heavy and light chain genes. All of the CDRs of a particular human antibody may be replaced with at least a portion of a non-human CDR, or only some of the CDRs may be replaced with non-human CDRs.

Those skilled in the art will be familiar with various methods of producing antibody fragments. For example, a Fab may be produced by treating an antibody with papaine, or by expressing both chains of the Fab in a prokaryotic or eukaryotic cell. A F(ab')2 may be produced by treating an antibody with pepsin, or by binding Fab' via a thioether or a disulfide bond. On the other hand, a Fab' can be produced by treating F(ab')2 with a reducing agent such as dithiothreitol (DTT), or by expressing the Fab' chains in a prokaryotic or eukaryotic cell. An scFv may be produced by expressing the CDRs or VH and VL domains in a prokaryotic or eukaryotic cell. CDR grafting may also be employed to generate a humanised scFv fragment. A single chain antibody or VHH may be generated by immunising a Camelidae mammal with the target antigen (eg, TM6SF2), taking a sample from the immunised Camelidae mammal and isolating heavy chain antibody sequences and/or VHH sequences directed against the target antigen. Single chain antibodies or VHH may also be produced by screening a library comprising heavy chain antibody sequences and/or VHH sequences. Other methods of producing antibody fragments will be well known by those skilled in the art.

Antibodies and fragments therefore may be engineered to increase their ability to enter cells and target intracellular TM6SF2. Suitable techniques for enhancing intracellular activity of an antibody or a fragment thereof are known by those skilled in the art and include the techniques described by Trenevska et al. (2017) Frontiers in Immunology, 8: 1001.

### Compositions and methods

The present disclosure provides methods of treating a hepatitis B virus infection in a subject the method comprising administering to the subject an inhibitor wherein the inhibitor inhibits or suppresses a regulator of liver lipid metabolism in the subject. The compositions disclosed in the present applications are for use in the methods of treating as described herein. The regulator may regulate lipid synthesis and/or lipid metabolism in the subject. The regulator may regulate cholesterol synthesis and/or lipoprotein secretion in the liver. Preferably, the regulator is TM6SF2. As described herein, administration of the inhibitor preferably reduces HBsAg concentration in serum of the subject. Accordingly, the present disclosure also provides methods of reducing the concentration of HBsAg in serum of a subject the method comprising administering to the subject an inhibitor of TM6SF2. Also provided are methods of treating a hepatitis D virus infection in a subject the method comprising administering to the subject an inhibitor of TM6SF2.

In certain embodiments, the present disclosure provides a method of treating a hepatitis B virus infection in a subject the method comprising administering to the subject an inhibitor of TM6SF2 wherein the inhibitor of TM6SF2 is a nucleic acid comprising at least 20 contiguous nucleotides which are substantially complementary to a sequence within TM6SF2 mRNA (SEQ ID NO. 3), wherein the nucleic acid is between 20 and 50 nucleotides in length. In certain embodiments, the present disclosure provides a method of treating a hepatitis B virus infection in a subject the method comprising administering to the subject an inhibitor of TM6SF2 wherein the inhibitor of TM6SF2 is an RNA comprising at least 20 contiguous nucleotides which are substantially complementary to the nucleotide sequence set forth in SEQ ID NO. 3, wherein the RNA is between 20 and 50 nucleotides in length. In certain embodiments, the present disclosure provides a method of treating a hepatitis B virus infection in a subject the method comprising administering to the subject an inhibitor of TM6SF2 wherein the inhibitor of TM6SF2 is an siRNA or a vector encoding an siRNA comprising at least 20 contiguous nucleotides which are substantially complementary to the nucleotide sequence set forth in SEQ ID NO. 3, wherein the siRNA is between 20 and 30 nucleotides in length. In certain embodiments, the present disclosure provides a method of treating a hepatitis B virus infection in a subject the method comprising administering to the subject an inhibitor of TM6SF2 wherein the inhibitor of TM6SF2 is an siRNA or a vector encoding an siRNA comprising at least 20 contiguous nucleotides which are substantially complementary to the nucleotide sequence set forth in SEQ ID NO. 3, wherein the siRNA is between 20 and 30 nucleotides in length, and wherein the siRNA or the vector is conjugated to N-acetylgalactosamine. In certain embodiments, the present disclosure provides a method of treating a hepatitis B virus infection in a subject the method comprising administering to the subject an inhibitor of TM6SF2 wherein the inhibitor of TM6SF2 is a nucleic acid comprising at least 20 contiguous nucleotides which are at least 80% identical to a nucleotide sequence set forth in SEQ ID NO 4, wherein the nucleic acid is between 20 and 50 nucleotides in length. In certain embodiments, the present disclosure provides a method of treating a hepatitis B virus infection in a subject the method comprising administering to the subject an inhibitor of TM6SF2 wherein the inhibitor of TM6SF2 is an siRNA or a vector encoding an siRNA comprising at least 20 contiguous nucleotides which are at least 80% identical to a nucleotide sequence set forth in SEQ ID NO 4, wherein the siRNA is between 20 and 30 nucleotides in length. In certain embodiments, the present disclosure provides a method of treating a hepatitis B virus infection in a subject the method comprising administering to the subject an inhibitor of TM6SF2 wherein the inhibitor of TM6SF2 is an antibody or a fragment thereof. In certain embodiments, the present disclosure provides a method of treating a hepatitis B virus infection in a subject the method comprising administering to the subject an inhibitor of TM6SF2 wherein the inhibitor of TM6SF2 is a small organic molecule.

The present disclosure also provides methods of reducing the concentration of HBsAg in serum of a subject the method comprising administering to the subject an inhibitor of TM6SF2 as described herein. Accordingly, in certain embodiments, the present disclosure provides a method of reducing the concentration of HBsAg in serum of a subject the method comprising administering to the subject an inhibitor of TM6SF2 wherein the inhibitor of TM6SF2 is a nucleic acid comprising at least 20 contiguous nucleotides which are substantially complementary to a sequence within TM6SF2 mRNA (SEQ ID NO. 3), wherein the nucleic acid is between 20 and 50 nucleotides in length. In certain embodiments, the present disclosure provides a method of reducing the concentration of HBsAg in serum of a subject the method comprising administering to the subject an inhibitor of TM6SF2 wherein the inhibitor of TM6SF2 is an RNA comprising at least 20 contiguous nucleotides which are substantially complementary to the nucleotide sequence set forth in SEQ ID NO. 3, wherein the RNA is between 20 and 50 nucleotides in length. In certain embodiments, the present disclosure provides a method of reducing the concentration of HBsAg in serum of a subject the method comprising administering to the subject an inhibitor of TM6SF2 wherein the inhibitor of TM6SF2 is an siRNA or a vector encoding an siRNA comprising at least 20 contiguous nucleotides which are substantially complementary to the nucleotide sequence set forth in SEQ ID NO. 3, wherein the siRNA is between 20 and 30 nucleotides in length. In certain embodiments, the present disclosure provides a method of reducing the concentration of HBsAg in serum of a subject the method comprising administering to the subject an inhibitor of TM6SF2 wherein the inhibitor of TM6SF2 is an siRNA or a vector encoding an siRNA comprising at least 20 contiguous nucleotides which are substantially complementary to the nucleotide sequence set forth in SEQ ID NO. 3, wherein the siRNA is between 20 and 30 nucleotides in length, and wherein the siRNA or the vector is conjugated to N-acetylgalactosamine. In certain embodiments, the present disclosure provides a method of reducing the concentration of HBsAg in serum of a subject the method comprising administering to the subject an inhibitor of TM6SF2 wherein the inhibitor of TM6SF2 is a nucleic acid comprising at least 20 contiguous nucleotides which are at least 80% identical to a nucleotide sequence set forth in SEQ ID NO 4, wherein the nucleic acid is between 20 and 50 nucleotides in length. In certain embodiments, the present disclosure provides a method of reducing the concentration of HBsAg in serum of a subject the method comprising administering to the subject an inhibitor of TM6SF2 wherein the inhibitor of TM6SF2 is an siRNA or a vector encoding an siRNA comprising at least 20 contiguous nucleotides which are at least 80% identical to a nucleotide sequence set forth in SEQ ID NO 4, wherein the siRNA is between 20 and 30 nucleotides in length. In certain embodiments, the present disclosure provides a method of reducing the concentration of HBsAg in serum of a subject the method comprising administering to the subject an inhibitor of TM6SF2 wherein the inhibitor of TM6SF2 is an antibody or a fragment thereof. In certain embodiments, the present disclosure provides a method of reducing the concentration of HBsAg in serum of a subject the method comprising administering to the subject an inhibitor of TM6SF2 wherein the inhibitor of TM6SF2 is a small organic molecule.

The present disclosure also provides methods of treating a hepatitis D virus infection in a subject the method comprising administering to the subject an inhibitor of TM6SF2 as described herein. Accordingly, in certain embodiments, the present disclosure provides a method of treating a hepatitis D virus infection in a subject the method comprising administering to the subject an inhibitor of TM6SF2 wherein the inhibitor of TM6SF2 is a nucleic acid comprising at least 20 contiguous nucleotides which are substantially complementary to a sequence within TM6SF2 mRNA (SEQ ID NO. 3), wherein the nucleic acid is between 20 and 50 nucleotides in length. In certain embodiments, the present disclosure provides a method of treating a hepatitis D virus infection in a subject the method comprising administering to the subject an inhibitor of TM6SF2 wherein the inhibitor of TM6SF2 is an RNA comprising at least 20 contiguous nucleotides which are substantially complementary to the nucleotide sequence set forth in SEQ ID NO. 3, wherein the RNA is between 20 and 50 nucleotides in length. In certain embodiments, the present disclosure provides a method of treating a hepatitis D virus infection in a subject the method comprising administering to the subject an inhibitor of TM6SF2 wherein the inhibitor of TM6SF2 is an siRNA or a vector encoding an siRNA comprising at least 20 contiguous nucleotides which are substantially complementary to the nucleotide sequence set forth in SEQ ID NO. 3, wherein the siRNA is between 20 and 30 nucleotides in length. In certain embodiments, the present disclosure provides a method of treating a hepatitis D virus infection in a subject the method comprising administering to the subject an inhibitor of TM6SF2 wherein the inhibitor of TM6SF2 is an siRNA or a vector encoding an siRNA comprising at least 20 contiguous nucleotides which are substantially complementary to the nucleotide sequence set forth in SEQ ID NO. 3, wherein the siRNA is between 20 and 30 nucleotides in length, and wherein the siRNA or the vector is conjugated to N-acetylgalactosamine. In certain embodiments, the present disclosure provides a method of treating a hepatitis D virus infection in a subject the method comprising administering to the subject an inhibitor of TM6SF2 wherein the inhibitor of TM6SF2 is a nucleic acid comprising at least 20 contiguous nucleotides which are at least 80% identical to a nucleotide sequence set forth in SEQ ID NO 4, wherein the nucleic acid is between 20 and 50 nucleotides in length. In certain embodiments, the present disclosure provides a method of treating a hepatitis D virus infection in a subject the method comprising administering to the subject an inhibitor of TM6SF2 wherein the inhibitor of TM6SF2 is an siRNA or a vector encoding an siRNA comprising at least 20 contiguous nucleotides which are at least 80% identical to a nucleotide sequence set forth in SEQ ID NO 4, wherein the siRNA is between 20 and 30 nucleotides in length. In certain embodiments, the present disclosure provides a method of treating a hepatitis D virus infection in a subject the method comprising administering to the subject an inhibitor of TM6SF2 wherein the inhibitor of TM6SF2 is an antibody or a fragment thereof. In certain embodiments, the present disclosure provides a method of treating a hepatitis D virus infection in a subject the method comprising administering to the subject an inhibitor of TM6SF2 wherein the inhibitor of TM6SF2 is a small organic molecule.

The TM6SF2 inhibitors of the present disclosure may be administered as a composition which includes materials for increasing the biological stability of the TM6SF2 inhibitor and/or materials that increase the ability of the composition to penetrate hepatocytes selectively. The TM6SF2 inhibitor is preferably administered with a pharmaceutically acceptable carrier (eg, physiological saline), which is selected on the basis of the mode and route of administration, and standard pharmaceutical practice. In some embodiments, an isotonic formulation is used. Generally, additives for isotonicity can include sodium chloride, dextrose, mannitol, sorbitol and lactose. In some cases, isotonic solutions such as phosphate buffered saline are preferred. Stabilizers include gelatin and albumin. In some examples, a vasoconstriction agent is added to the formulation. The compositions according to the present disclosure are preferably sterile and pyrogen free.

Local modes of administration include intraparenchymal delivery to the liver, intrahepatic artery infusion and infusion into the portal vein. In certain embodiments, significantly smaller amounts of the components may exert an effect when administered locally (for example, directly into the liver parenchyma) compared to when administered systemically (for example, intravenously). Local modes of administration may reduce or eliminate the incidence of potentially toxic side effects that may occur when therapeutically effective amounts of a component are administered systemically.

Parenteral routes include, by way of example, intravenous, intrarterial, intramuscular, intradermal, subcutaneous, intranasal, and intraperitoneal routes. Targeted transfection of hepatocytes in vivo may be accomplished through IV injection with a composition comprising a TM6SF2 inhibitor as described herein complexed with a mixture (eg, a 35%:65% ratio) of a lactosyl spermine (mono or trilactosylated) and cholesteryl spermine (containing spermine to DNA at a charge ratio of 0.8). Such compositions are useful for pharmaceutical applications and may readily be formulated in a suitable sterile, non-pyrogenic vehicle, eg, buffered saline for injection, for parenteral administration, eg, IV (including IV infusion), IM, SC, and for intraperitoneal administration. In certain compositions, the TM6SF2 inhibitor of the disclosure is complexed with an endosomolytic spermine such as cholesteryl spermine alone, without a targeting spermine; some routes of administration, such as intraperitoneal injection or infusion, may achieve effective hepatic delivery and transfection. Intraperitoneal administration (eg, ultrasound guided intraperitoneal injection) of a sterile pharmaceutical composition comprising a TM6SF2 inhibitor of the present disclosure in a specially formulated delivery vehicle may be an advantageous route of delivery to promote uptake by liver cells, including hepatocytes. The volume, concentration, and formulation of the pharmaceutical composition as well as the dosage regimen may be tailored specifically to maximize cellular delivery while minimizing toxicity such as an inflammatory response. For example, relatively large volumes (5, 10, 20, 50 ml or more) with corresponding low concentrations of active ingredients, as well as the inclusion of an antiinflammatory compound such as a corticosteroid, may be utilized if desired.

Administration may be provided as a periodic bolus (for example, intravenously) or as continuous infusion from an internal reservoir or from an external reservoir (for example, from an intravenous bag or implantable pump). Components may be administered locally, for example, by continuous release from a sustained release drug delivery device implanted in the liver.

In addition, components may be formulated to permit release over a prolonged period of time. A release system can include a matrix of a biodegradable material or a material which releases the incorporated components by diffusion. The components can be homogeneously or heterogeneously distributed within the release system. A variety of release systems may be useful, however, the choice of the appropriate system will depend upon rate of release required by a particular application. Both non-degradable and degradable release systems can be used. Suitable release systems include polymers and polymeric matrices, non-polymeric matrices, or inorganic and organic excipients and diluents such as, but not limited to, calcium carbonate and sugar (for example, trehalose). The release system material can be selected so that components having different molecular weights are released by diffusion or through degradation of the material. Representative synthetic, biodegradable polymers include, for example: polyamides such as poly(amino acids) and poly(peptides); polyesters such as poly(lactic acid), poly(glycolic acid), poly(lactic-co-glycolic acid), and poly(caprolactone); poly(anhydrides); polyorthoesters; polycarbonates; and chemical derivatives thereof (substitutions, additions of chemical groups, for example, alkyl, alkylene, hydroxylations, oxidations, and other modifications routinely made by those skilled in the art), copolymers and mixtures thereof. Representative synthetic, non-degradable polymers include, for example: polyethers such as poly(ethylene oxide), poly(ethylene glycol), and poly(tetramethylene oxide); vinyl polymers-polyacrylates and polymethacrylates such as methyl, ethyl, other alkyl, hydroxyethyl methacrylate, acrylic and methacrylic acids, and others such as poly(vinyl alcohol), poly(vinyl pyrolidone), and poly(vinyl acetate); poly(urethanes); cellulose and its derivatives such as alkyl, hydroxyalkyl, ethers, esters, nitrocellulose, and various cellulose acetates; polysiloxanes; and any chemical derivatives thereof (substitutions, additions of chemical groups, for example, alkyl, alkylene, hydroxylations, oxidations, and other modifications routinely made by those skilled in the art), copolymers and mixtures thereof. Poly(lactide-co-glycolide) microspheres can also be used.

Dosages may vary with the type and severity of the condition to be treated, and may include single or multiple dosses. Specific dosage regimens may be adjusted over time according to the individual need and the professional judgment of the practitioner administering the composition. When administered to a human subject, the dosage regimen may vary depending on a variety of factors including the type and severity of the condition, the age, sex, weight or medical condition of the subject and the route of administration. In that regard, precise amounts of the TM6SF2 inhibitor to be administered will depend on the judgement of the practitioner.

The compositions described herein may be administered over a period of hours, days, weeks, or months, depending on several factors, including the severity of the condition being treated, whether a recurrence is considered likely, etc. The administration may be constant, eg, constant infusion over a period of hours, days, weeks, months, etc. Alternatively, the administration may be intermittent, eg, once per day over a period of days, once per hour over a period of hours, or any other such schedule as deemed suitable.

In certain embodiments, the TM6SF2 inhibitor may be administered to a subject at a dose of about 0.05 mg/m², 0.1 mg/m², 0.5 mg/m², 1 mg/m², 2 mg/m², 5 mg/m², 12 mg/m², 15 mg/m², 18 mg/m², 20 mg/m², 25 mg/m², 30 mg/m², 35 mg/m², 40 mg/m², 50 mg/m², 60 mg/m², 70 mg/m², 80 mg/m², 90 mg/m², 100 mg/m², 110 mg/m², 120 mg/m², 130 mg/m², 140 mg/m², 150 mg/m², 160 mg/m², 170 mg/m², 180 mg/m², 190 mg/m², 200 mg/m², 210 mg/m², 220 mg/m², 230 mg/m², 240 mg/m², 250 mg/m², 260 mg/m², 270 mg/m², 280 mg/m², 290 mg/m², 300 mg/m², 310 mg/m², 320 mg/m², 330 mg/m², 340 mg/m², 350 mg/m², 360 mg/m², 370 mg/m², 380 mg/m², 390 mg/m², 400 mg/m², 410 mg/m², 420 mg/m², 430 mg/m², 440 mg/m², 450 mg/m², 460 mg/m², 470 mg/m², 480 mg/m², 490 mg/m², 500 mg/m² or more. In embodiments where the inhibitor of TM6SF2 is nucleic acid, a suitable dose may be in the range of about 0.001 to about 200.0 milligrams per kilogram body weight of the recipient per day, generally in the range of about 1 to 50 mg per kilogram body weight per day. For example, the nucleic acid may be administered at about 0.01 mg/kg, about 0.05 mg/kg, about 0.5 mg/kg, about 1 mg/kg, about 1.5 mg/kg, about 2 mg/kg, about 3 mg/kg, about 10 mg/kg, about 20 mg/kg, about 30 mg/kg, about 40 mg/kg, or about 50 mg/kg per dose.

In certain embodiments, the methods of the present disclosure comprise initiating treatment of a subject in an advanced stage of disease, eg, during immune-tolerant phase, eg, during immune-active phase, eg, during inactive carrier phase. In certain embodiments, the method comprises initiating treatment of a subject in the case of acute disease. In certain embodiments, the method comprises initiating treatment of a subject in the case of severe disease exacerbation, eg, during acute hepatitis. In certain embodiments, the method comprises initiating treatment of a subject in the case of asymptomatic disease, eg, during latent infection, eg, during chronic infection with low ALT levels and/or low HBV DNA levels and/or absence of cirrhosis.

In certain embodiments, the method comprises initiating treatment of a subject at risk for hepatocellular carcinoma secondary to exposure to acute HBV. In certain embodiments, the method comprises initiating treatment of a subject at risk for hepatocellular carcinoma due to chronic HBV. In certain embodiments, the method comprises initiating treatment of a subject at risk for hepatocellular carcinoma due to exposure to HBV, including but not limited to subjects with increased HBV integration events, subjects with HBV integration events in known oncogenes, subjects with HBV integration events in exonic and/or promoter regions. In certain embodiments, the method comprises initiating treatment of a subject prior to disease expression. In certain embodiments, the method comprises initiating treatment of a subject in an early stage of disease, eg, when a subject has been exposed to HBV or is thought to have been exposed to HBV. In certain embodiments, the method comprises initiating treatment of a subject at the appearance of elevated liver enzymes, eg, elevated AST or elevated ALT. In certain embodiments, the method comprises initiating treatment at the appearance of any of the following symptoms consistent or associated with HBV infection: jaundice, nausea and vomiting, weakness, dark urine, fever, abdominal pain, loss of appetite, confusion and changes in mental status, joint pain, spider angioma, palmar erythema, hepatomegaly, jaundice, splenomegaly easy bruising and bleeding, hepatic encephalopathy or portal hypertension.

In certain embodiments, the compositions of the present disclosure further comprise an antiviral agent, and the methods of the present disclosure may comprise administering to a subject an inhibitor of TM6SF2 and an antiviral agent. Suitable antiviral agents may include interferon, such as pegylated interferon, nucleic acid polymers (see, eg, Vaillant (2016) Antiviral Res. 133: 32-40), nucleoside analogues such as lamivudine, entecavir, tenofovir and telbivudine, and nucleic acid molecules targeting HBV, such as HBV-specific siRNA, miRNA or antisense nucleic acid molecules. Other suitable antiviral agents may include cccDNA formation inhibitors, antiviral core protein mutant, HBc directed transbodies, RNAi agents targeting HBV RNA, immunostimulants, TLR-7/9 agonists, cyclophilin inhibitors, HBV vaccines, heteroaryldihydropyrimidines, HBV capsid inhibitors, SMAC mimetics, epigenetic modulators, kinase inhibitors, checkpoint inhibitors and STING agonists.

Suitable HBV capsid inhibitors may include 3-3[(8aS)-7-[[(4S)-5-ethoxycarbonyl-4-(3-fluoro-2-methyl-phenyl)-2-thiazol-2-yl-1,4dihydropyrimidin-6-yl]methyl]-3-oxo-5,6,8,8a-tetrahydro-1H-imidazo [1,5-a]pyrazin-2-yl]-2,2dimethyl-propanoic acid; 3-[(8aS)-7-[[(4R)-4-(2-chloro-4-fluoro-phenyl)-5-methoxycarbonyl-2-thiazol-2-yl-1,4-dihydropyrimidin-6-yl]methyl]-3-oxo-5,6,8,8a-tetrahydro-1H-imidazo[1,5-a]pyrazin-2-yl]-2,2-dimethyl-propanoic acid; 3-[(8aS)-7-[[(4R)-4-(2-cbloro-3-fluoro-phenyl)-5-ethoxycarbonyl-2-thiazol-2-yl-1,4-dihydropyrimidin-6-yl]methyl]-3-oxo-5,6,8,8a-tetrahydro-1H-imidazo[1,5-a]pyrazin-2-yl]-2,2-dimethyl-propanoic acid; 4-[(8aS)-7-[[(4R)-4-(2-chloro-4-fluoro-phenyl)-5-methoxycarbonyl-2-thiazol-2-yl-1,4-dihydropyrimidin-6-yl]methyl]-3-oxo-5,6,8,8a-tetrahydro-1H-imidazo[1,5-a]pyrazin-2-yl]-3,3-dimethyl-butanoic acid; (8S,8aR)-2-tert-butyl-7-[[(4R)-4-(2-chloro-3-fluoro-phenyl)-5-ethoxycarbonyl-2-thiazol-2-yl-1,4-dihydropyrimidin-6-yl]methyl]-3-oxo-5,6,8,8a-tetrahydro-1H-imidazo[1,5-a]pyrazine-8-carboxylic acid; or (8R,8aS)-2-tert-butyl-7-[[(4R)-4-(2-chloro-3-fluoro-phenyl)-5-ethoxycarbonyl-2-thiazol-2-yl-1,4-dihydropyrimidin-6-yl]methyl]-3-oxo-5,6,8,8a-tetrahydro-1H-imidazo[1,5-a]pyrazine-8-carboxylic acid. Other HBV capsid inhibitors may include Bay 41-4109, Bay 38-7690, Bay 39-5493, GLS4, AT-61 and AT-130. Other HBV capsid inhibitors include those disclosed in WO2017/076791, WO2015/132276, WO2008/154819, WO2014/029193, WO2015/074546, CN103664897 and CN103664925.

Suitable interferons may include interferon alfa, beta or gamma known to those skilled in the art, such as, interferon alfa-n1 (eg, Surniferon^{®}, Sumitomo^{®}), interferon alfa-n3, interferon alfa-2a (Roferon A^{®}, Hoffmann-La Roche, Inc.) interferon alfa-2b (Intron A^{®}, Schering-Plough Corp.), interferon alfa-2c (Berofor^{®}, Boehringer Ingelheim, Inc.) and consensus interferon (Infergen^{®}, InterMune, Inc.). The interferon may be non-conjugated inteferon or a pegylated interferon.

In certain embodiments, the antiviral agent is selected from one of the following: HBV caspid assembly modulators (eg, GLS4, BAY 41-4109, AT-130, DVR-23), other CpAMs such as those disclosed in WO2014/037480, WO2014/184328, WO2013/006394, WO2014/089296, WO2014/106019, WO2013/102655, WO2014/184350, WO2014/184365, WO2014/161888, WO2014/131847, WO2014/033176, WO2014/033167 and WO2014/033170; nucleoside analogs interfering with viral polymerase, such as entecavir (Baraclude), Lamivudine, (Epivir-HBV), Telbivudine (Tyzeka, Sebivo), Adefovir dipivoxil (Hepsera), Tenofovir (Viread), Tenofovir alafenamide fumarate (TAF), prodrugs of tenofavir (eg AGX-1009), L-FMAU (Clevudine), LB80380 (Besifovir) and viral entry inhibitors such as Myrcludex B and related lipopeptide derivatives; HBsAg secretion inhibitors such as REP 9AC' and related nucleic acid-based amphipathic polymers, HBF-0529 (PBHBV-001), PBHBV-2-15, disruptors of nucleocapsid formation or integrity such as NZ-4/W28F; cccDNA formation inhibitors such as BSBI-25, CCC-0346, CCC-0975; HBc-directed transbodies such as those described by Wang et al. (2014) Int. Immunopharmacol.*;* antiviral core protein mutant (such as Cp183-V124W and related mutations as described in WO2013/010069 and WO2014/074906); inhibitors of HBx-interactions such as RNAi, antisense and nucleic acid based polymers targeting HBV RNA;, eg, RNAi (for example ALN-HBV, ARC-520, TKM-HBV, ddRNAi), antisense (ISIS-HBV), or nucleic acid based polymer: (REP 2139-Ca); immunostimulants such as Interferon alpha 2a (Roferon), Intron A (interferon alpha 2b), Pegasys (peginterferon alpha 2a), pegylated IFN 2b, IFN lambda Ta and PEG IFN lambda Ta, Wellferon, Roferon, Infergen, lymphotoxin beta agonists such as CBE1 1 and BS1); non-interferon Immune enhancers such as thymosin alpha-1 (Zadaxin) and interleukin-7 (CYT107); TLR-7/9 agonists such as GS-9620, CYT003, Resiquimod; cyclophilin inhibitors such as NVP018; OCB-030; SCY-635; Alisporivir; NIM811 and related cyclosporine analogs; vaccines such as GS-4774, TG1050, core antigen vaccine; SMAC mimetics such as birinapant and other IAP-antagonists; epigenetic modulators such as KMT inhibitors (EZH1/2, G9a, SETD7, Suv39 inhibitors), PRMT inhibitors, HDAC inhibitors, SIRT agonists, HAT inhibitors, WD antagonists (eg OICR-9429), PARP inhibitors, APE inhibitors, DNMT inhibitors, LSD1 inhibitors, JMJD HDM inhibitors and bromodomain antagonists; kinase inhibitors such as TKB1 antagonists, PLK inhibitors, SRPK inhibitors, CDK2 inhibitors, ATM & ATR kinase inhibitors; STING agonists; ribavirin; N-acetyl cysteine ; NOV-205 (BAM205); nitazoxanide (Alinia), tizoxanide; SB 9200 Small Molecule Nucleic Acid Hybrid (SMNH); DV-601; arbidol; FXR agonists (such as GW 4064 and Fexaramin); antibodies, therapeutic proteins, gene therapy, and biologics directed against viral components or interacting host proteins.

In preferred embodiments, the antiviral agent suppresses accumulation of HBV DNA in the subject. In that way, the antiviral agent complements the TM6SF2 inhibitor which suppresses HBsAg secretion in the subject. In preferred embodiments, the antiviral agent is an interferon or a nucleoside analogue. For example, the antiviral agent may be pegylated interferon. In certain embodiments, the antiviral agent is entecavir or tenofovir.

In certain embodiments, the present disclosure provides a composition comprising: an inhibitor of TM6SF2; an antiviral agent; and a pharmaceutically acceptable carrier. For example, the present disclosure provides a composition comprising: an inhibitor of TM6SF2; an interferon or a nucleoside analogue; and a pharmaceutically acceptable carrier. For example, the present disclosure provides a composition comprising: an inhibitor of TM6SF2; pegylated interferon; and a pharmaceutically acceptable carrier. For example, the present disclosure provides a composition comprising: an inhibitor of TM6SF2; an antiviral agent such as an interferon or a nucleoside analogue; and a pharmaceutically acceptable carrier wherein the inhibitor of TM6SF2 is a nucleic acid comprising at least 20 contiguous nucleotides which are substantially complementary to a sequence within TM6SF2 mRNA (SEQ ID NO. 3), wherein the nucleic acid is between 20 and 50 nucleotides in length. For example, the present disclosure provides a composition comprising: an inhibitor of TM6SF2; an antiviral agent such as an interferon or a nucleoside analogue; and a pharmaceutically acceptable carrier wherein the inhibitor of TM6SF2 is an RNA comprising at least 20 contiguous nucleotides which are substantially complementary to the nucleotide sequence set forth in SEQ ID NO. 3, wherein the RNA is between 20 and 50 nucleotides in length. For example, the present disclosure provides a composition comprising: an inhibitor of TM6SF2; an antiviral agent such as an interferon or a nucleoside analogue; and a pharmaceutically acceptable carrier wherein the inhibitor of TM6SF2 is an siRNA or a vector encoding an siRNA comprising at least 20 contiguous nucleotides which are substantially complementary to the nucleotide sequence set forth in SEQ ID NO. 3, wherein the siRNA is between 20 and 30 nucleotides in length. For example, the present disclosure provides a composition comprising: an inhibitor of TM6SF2; an antiviral agent such as an interferon or a nucleoside analogue; and a pharmaceutically acceptable carrier wherein the inhibitor of TM6SF2 is a nucleic acid comprising at least 20 contiguous nucleotides which are at least 80% identical to a nucleotide sequence set forth in SEQ ID NO 4, wherein the nucleic acid is between 20 and 50 nucleotides in length. For example, the present disclosure provides a composition comprising: an inhibitor of TM6SF2; an antiviral agent such as an interferon or a nucleoside analogue; and a pharmaceutically acceptable carrier wherein the inhibitor of TM6SF2 is an siRNA or a vector encoding an siRNA comprising at least 20 contiguous nucleotides which are at least 80% identical to a nucleotide sequence set forth in SEQ ID NO 4, wherein the siRNA is between 20 and 30 nucleotides in length, and wherein the siRNA or the vector is conjugated to N-acetylgalactosamine. For example, the present disclosure provides a composition comprising: an inhibitor of TM6SF2; an antiviral agent such as an interferon or a nucleoside analogue; and a pharmaceutically acceptable carrier wherein the inhibitor of TM6SF2 is an antibody or a fragment thereof. For example, the present disclosure provides a composition comprising: an inhibitor of TM6SF2; an antiviral agent such as an interferon or a nucleoside analogue; and a pharmaceutically acceptable carrier wherein the inhibitor of TM6SF2 is a small organic molecule.

The antiviral agent and the inhibitor of TM6SF2 may be administered to the subject in separate compositions or in the same composition. In certain embodiments, the antiviral agent and the inhibitor of TM6SF2 are administered to the subject sequentially or simultaneously.

The present disclosure also provides methods of diagnosing susceptibility to hepatitis B virus infection in a subject the method comprising detecting the presence of a genetic polymorphism such as a SNP in TM6SF2 from a nucleic acid sample obtained from the subject. Subjects carrying a SNP which reduces the activity of TM6SF2, or which reduces the expression of TM6SF2, may be diagnosed as having a low susceptibility to a hepatitis B virus infection. For example, the method may detect an rs58542926 (C→T) SNP, in which case, the subject may be diagnosed as having a low susceptibility to hepatitis B virus infection. On the other hand, subjects carrying a SNP which increases the activity of TM6SF2, or which increases the expression of TM6SF2, may be diagnosed as having a high susceptibility to a hepatitis B virus infection. Other SNPs associated with TM6SF2 that may be detected in the presently disclosed methods include rs735273, rs998732, rs2074298, rs2074299, rs2074300, rs2074301, rs2074302, rs2074303, rs2074304, rs2074305, rs2074306, rs2074307, rs4319875, rs4503924, rs4808936, rs8103496, rs8112245, rs10419245, rs10419998, rs10422331, rs10422620, rs10426780, rs10623726, rs11669578, rs11669582, rs11670683, rs12459455, rs12459457, rs12461171, rs12974359, rs13344498, rs34370231, rs34372903, rs34746453, rs34766030, rs35176384, rs35854118, rs55690765, rs55822665, rs55877107, rs56831097, rs57002012, rs58409825, rs60518933, rs62135548, rs66543857, rs68050962, rs72999053, rs72999058, rs72999059, rs72999063, rs72999068, rs74182629, rs74182630, rs75776197, rs76522543, rs77113415, rs77355543, rs77495717, rs78009375, rs78386089, rs78538967, rs78640921, rs79300864, rs79526739, rs79552617, rs80059465, rs111364160, rs111630044, rs111987320, rs112534888, rs112783153, rs113046036, rs113379443, rs114212391, rs114319770, rs115439672, rs115589470, rs115679832, rs116904583, rs116956307, rs117182255, rs117284756, rs117846705, rs117877390, rs117957107, rs138026667, rs138159476, rs138370840, rs139343880, rs140260057, rs140881283, rs141184770, rs141568742, rs142024741, rs142056540, rs142207444, rs142859954, rs142906037, rs143372544, rs143598956, rs144418687, rs144821371, rs145901555, rs146079855, rs146121815, rs146784372, rs146850861, rs146985614, rs148169856, rs148205248, rs148883382, rs148937842, rs149250975, rs150177500, rs150904632, rs150912512, rs151226989, rs181300353, rs181728218, rs182033618, rs182231816, rs182431997, rs182617406, rs182848497, rs183516352, rs183882541, rs183980805, rs184107342, rs184202121, rs184254301, rs184613047, rs185101196, rs185174399, rs185295247, rs185424927, rs185956912, rs186541489, rs186811910, rs187156613, rs187429064, rs188201600, rs188445368, rs188755038, rs188787025, rs188841337, rs189627178, rs189644057, rs189891866, rs190164983, rs190419475, rs190427537, rs191420244, rs191671030, rs191881625, rs192042078, rs192578237, rs192880850, rs193215236, rs199661804, rs199729439, rs199729685, rs199926770, rs199962010, rs199996201, rs200095788, rs200096419, rs200148631, rs200149999, rs200162798, rs200174137, rs200392909, rs200475076, rs200492531, rs200494594, rs200570958, rs200633268, rs200672499, rs200709395, rs200817931, rs200962751, rs201123773, rs201138158, rs201189528, rs201198499, rs201350671, rs201385194, rs201510558, rs201552850, rs201595252, rs201639037, rs201727409, rs201830619, rs201880285, rs202115762, rs202188864, rs202196626, rs202224896, rs202232104, rs267605373, rs267605374, rs367550390, rs367611657, rs368032431, rs368135661, rs368169036, rs368179739, rs368432017, rs368449833, rs368592065, rs368696132, rs368893203, rs369198322, rs369248616, rs369399709, rs369732627, rs370140623, rs370326434, rs370349813, rs370418517, rs370548010, rs370811816, rs370960454, rs370996752, rs371140517, rs371256236, rs371788568, rs371796166, rs371811399, rs371823523, rs371882936, rs371973720, rs372372827, rs372429680, rs372610360, rs372689252, rs373173558, rs373418560, rs373585910, rs373609155, rs373723689, rs374051571, rs374302893, rs374568040, rs374675893, rs374741086, rs374852151, rs375486001, rs375522420, rs375598960, rs375651812, rs375759405, rs375976030, rs375988924, rs376400508, rs376664395, rs376668139, rs376713609, rs376854548, rs376977369, rs377761139, rs386388698, rs386807528, rs386807529, rs386807530, rs386807531, rs398034192, rs527514985, rs527605147, rs527809040, rs527986412, rs528120627, rs528169718, rs528181849, rs528647295, rs528694697, rs528780612, rs528930903, rs529952177, rs530116167, rs530136094, rs530320836, rs530488860, rs530509632, rs530522031, rs530648844, rs531509367, rs531644199, rs531835571, rs532107426, rs532282582, rs532385541, rs532447583, rs532717829, rs532776739, rs532961952, rs532974796, rs532980253, rs533619940, rs534030651, rs534362393, rs534415108, rs534503635, rs534583840, rs534982327, rs535018065, rs535260160, rs535543205, rs535554849, rs535612648, rs536329640, rs536372726, rs536426725, rs536607115, rs536617790, rs536924825, rs536926633, rs537127422, rs537425840, rs538527740, rs538720932, rs538738933, rs538850519, rs539351891, rs539755943, rs539818973, rs540371480, rs540634411, rs540647875, rs541393499, rs541575771, rs541723104, rs541971412, rs542724791, rs542782300, rs543185206, rs543204933, rs543742200, rs543742518, rs543933980, rs543959857, rs544144326, rs544510460, rs544964882, rs545019311, rs545153910, rs545530289, rs545890023, rs545949548, rs546163061, rs546295489, rs546522622, rs546529123, rs546809363, rs546932962, rs547034731, rs547208333, rs547292673, rs547413352, rs547519986, rs547769753, rs547798338, rs547824363, rs547862559, rs547910099, rs548135510, rs548388761, rs548707505, rs548804768, rs548927791, rs549048702, rs549344577, rs550015865, rs550069079, rs550263833, rs550326348, rs550374077, rs550539684, rs551308493, rs551445434, rs551711432, rs551749096, rs551774494, rs551882049, rs552298442, rs552369868, rs552447744, rs552549037, rs552753050, rs552937376, rs553116023, rs553263601, rs553463888, rs553868693, rs554134877, rs554147023, rs554165408, rs554245111, rs554335721, rs554408127, rs554478292, rs554613897, rs555080865, rs555299306, rs555364706, rs555561183, rs556016935, rs556750981, rs556872459, rs556931770, rs556940362, rs557082001, rs557462632, rs557596254, rs558195975, rs558322932, rs558395619, rs558530410, rs559055958, rs559421022, rs559498474, rs559559943, rs559669215, rs559686718, rs560226721, rs560746868, rs560882211, rs561261005, rs561279291, rs561534406, rs561681825, rs561791712, rs561940016, rs561951989, rs562465880, rs562712792, rs562812444, rs563334566, rs563377594, rs563703231, rs563710488, rs563794240, rs564109716, rs564176559, rs564512671, rs564726571, rs565154419, rs565323238, rs565409717, rs565609697, rs566271444, rs566658419, rs566722127, rs566746498, rs566926815, rs567164142, rs567672787, rs567841607, rs567845732, rs568092639, rs568224127, rs568888515, rs568975835, rs569607367, rs570112176, rs570320544, rs570334470, rs570464637, rs570511831, rs570677436, rs570837540, rs570876772, rs571390178, rs571461812, rs571593622, rs571657604, rs571923462, rs571970171, rs571990786, rs571991424, rs572592067, rs573118354, rs573135323, rs573142957, rs573802243, rs574141880, rs574320154, rs574806213, rs574851602, rs574999847, rs575273640, rs575480864, rs575867088, rs576866024, rs576994101, rs577020062, rs577084188, rs577205451, rs577282110, rs578098988, rs745375371, rs745379169, rs745718995, rs745753117, rs745940010, rs746000718, rs746054059, rs746238022, rs746292831, rs746297412, rs746544094, rs746573601, rs746675647, rs746704348, rs746847673, rs746913530, rs746938988, rs747114553, rs747172221, rs747376552, rs747547392, rs747581461, rs747904279, rs747954776, rs748008045, rs748156985, rs748219349, rs748225805, rs748235885, rs748280759, rs748506657, rs748636134, rs748791710, rs749049816, rs749092203, rs749099980, rs749132467, rs749156356, rs749255904, rs749356484, rs749424485, rs749478341, rs749479743, rs749630470, rs749644443, rs749736898, rs749897108, rs750028253, rs750038678, rs750563576, rs750614738, rs750715914, rs750848305, rs750852905, rs750920847, rs750981220, rs751109817, rs751177628, rs751180168, rs751294456, rs751370592, rs751484459, rs751556538, rs751648928, rs751699956, rs751717826, rs751924974, rs752005834, rs752044826, rs752074221, rs752203275, rs752303575, rs752314042, rs752359236, rs752381864, rs752479397, rs752530644, rs752646217, rs752650660, rs752827568, rs752926063, rs752976965, rs753204217, rs753263594, rs753282565, rs753394211, rs753434175, rs753487399, rs753726067, rs753777271, rs753819682, rs753830613, rs753868276, rs754008018, rs754061321, rs754290724, rs754359619, rs754524665, rs754540761, rs754636939, rs754722942, rs754780228, rs754831597, rs754880976, rs754901288, rs755034726, rs755256363, rs755279648, rs755297072, rs755563542, rs755644201, rs755952179, rs755952920, rs756047028, rs756222146, rs756458112, rs756508325, rs756776896, rs756814455, rs756923592, rs756971078, rs757078871, rs757245760, rs757260859, rs757537930, rs757561160, rs757603161, rs757648359, rs757719730, rs757743424, rs757767181, rs757770572, rs757823392, rs757898541, rs757962781, rs758178350, rs758285826, rs758380915, rs758465670, rs758530683, rs758547141, rs758699207, rs758792305, rs758849576, rs758933778, rs759105000, rs759157845, rs759303352, rs759316924, rs759361981, rs759405740, rs759458881, rs759726808, rs759781410, rs759875174, rs759899142, rs760093123, rs760187085, rs760224884, rs760256070, rs760361003, rs760439176, rs760443439, rs760708240, rs761018515, rs761028673, rs761122285, rs761313502, rs761384793, rs761821661, rs761847887, rs761875061, rs761921380, rs762013546, rs762223286, rs762533772, rs762584400, rs762607638, rs762649345, rs762681767, rs763138841, rs763197011, rs763226075, rs763289803, rs763346894, rs763626164, rs763650790, rs763806867, rs763896047, rs763937811, rs763989049, rs764092018, rs764151271, rs764166050, rs764335067, rs764347802, rs764373234, rs764523700, rs764525288, rs764677340, rs764687758, rs764738579, rs764796534, rs764843325, rs764896578, rs764997559, rs765124011, rs765307791, rs765308950, rs765487792, rs765539044, rs765587945, rs765718156, rs765868864, rs765870085, rs765874061, rs765934243, rs766055026, rs766109869, rs766168663, rs766393161, rs766450426, rs766513047, rs766591574, rs766769956, rs766900807, rs766962077, rs767020475, rs767088074, rs767198313, rs767294984, rs767336308, rs767437386, rs767558951, rs767646066, rs767690398, rs767777461, rs768109212, rs768178426, rs768286445, rs768295893, rs768411760, rs768412914, rs768457575, rs768472296, rs768634850, rs768680912, rs768743022, rs768810724, rs768892596, rs768967492, rs769014596, rs769099177, rs769438472, rs769568288, rs769723406, rs769776834, rs769835494, rs770149338, rs770159397, rs770207713, rs770302899, rs770345515, rs770392287, rs770439989, rs770485209, rs770652877, rs770917391, rs770917482, rs770924273, rs770996540, rs771039998, rs771146280, rs771294107, rs771340141, rs771388055, rs771433257, rs771442632, rs771505286, rs771722501, rs771774218, rs771796358, rs771905872, rs771910676, rs772045939, rs772104990, rs772129480, rs772142094, rs772230256, rs772467670, rs772632305, rs772662650, rs772747156, rs772854285, rs772915395, rs772980616, rs773047608, rs773047703, rs773048268, rs773383618, rs773534873, rs773586331, rs773666481, rs773853708, rs774069430, rs774124975, rs774190231, rs774425469, rs774434974, rs774592616, rs774644327, rs774977414, rs775024557, rs775074014, rs775200974, rs775286282, rs775305208, rs775461758, rs775506391, rs775674052, rs775760280, rs775768034, rs775787345, rs775791193, rs775824227, rs775897073, rs775934456, rs775952201, rs776157523, rs776239655, rs776255115, rs776356715, rs776551647, rs776921682, rs777035225, rs777094771, rs777098305, rs777214490, rs777325985, rs777369177, rs777456748, rs777516403, rs777652355, rs777707469, rs777751853, rs777950487, rs778167482, rs778181000, rs778218963, rs778220855, rs778373253, rs778414824, rs778506705, rs778727994, rs778785086, rs778834417, rs778983262, rs778992711, rs779141868, rs779164806, rs779316134, rs779400582, rs779666905, rs779929001, rs780079840, rs780116992, rs780127806, rs780173234, rs780216665, rs780447055, rs780511777, rs780590671, rs780617841, rs780882997, rs780974435, rs781249230, rs781508882, rs781510146, rs781563282, rs781597593, rs781640798, rs781681958, rs781763445, rs796544366, rs796629311, rs865779671, rs866016264, rs866029777, rs866079644, rs866325262, rs866542844, rs866614531, rs866872816, rs867126720, rs867582659, rs867763922, rs868152043, rs868177285, rs868234954, rs868342017, rs868404085, rs868414711, rs868594415, rs869074161, rs879143337, rs879755394, rs886434369, rs886544758, rs886590104, rs886618081, rs886897647, rs887474125, rs887654448, rs887890746, rs888435236, rs888594428, rs888658449, rs888939457, rs889721057, rs889975468, rs890092314, rs890137864, rs890180763, rs890289824, rs890983124, rs891398404, rs891438078, rs891452330, rs891608978, rs891613961, rs892314841, rs892478039, rs892517556, rs892603469, rs893133155, rs893185700, rs893300947, rs893487341, rs894526989, rs894802997, rs894834167, rs894985010, rs895838889, rs895994568, rs896111563, rs896153730, rs897367337, rs897424298, rs899342057, rs899454357, rs900117791, rs900170218, rs900235828, rs900262903, rs900331335, rs900685051, rs900849456, rs901005048, rs901722125, rs901805601, rs901968099, rs902003532, rs902169018, rs902282439, rs903077025, rs903128024, rs903171440, rs903199489, rs903228452, rs903478436, rs903910405, rs904179550, rs904485791, rs904743660, rs904795007, rs905525368, rs905670454, rs905828481, rs906020972, rs906924935, rs907357146, rs908136050, rs908208517, rs908861045, rs909034262, rs909062141, rs909175521, rs910021533, rs910034221, rs910066647, rs910194439, rs910255917, rs910564287, rs910680224, rs911570700, rs911725748, rs911777996, rs911795177, rs911839910, rs911884102, rs912573137, rs912812891, rs913187459, rs913318637, rs913349619, rs913986808, rs914054140, rs914345911, rs914655222, rs914775502, rs914827983, rs914952001, rs915059501, rs915194650, rs915597936, rs915856507, rs915859711, rs916070257, rs916374871, rs916408069, rs916537112, rs917433448, rs917527710, rs917534022, rs917692268, rs918807147, rs918845166, rs918896445, rs919035806, rs919641136, rs919895432, rs920197136, rs920681527, rs921548959, rs921712241, rs922241754, rs922478450, rs922636873, rs923084773, rs923334335, rs923385232, rs923416902, rs923594836, rs923616592, rs923673856, rs923751919, rs923812201, rs923891065, rs924590902, rs924634410, rs924649744, rs925588999, rs925794334, rs926281678, rs926286738, rs927106392, rs927137782, rs927251085, rs927530949, rs927551421, rs929175975, rs929187382, rs929203247, rs929299009, rs929317281, rs930152639, rs930253225, rs930345155, rs930411760, rs930826751, rs930967952, rs931138472, rs931317075, rs931318388, rs931663524, rs931694744, rs931859288, rs932146567, rs932886781, rs932982597, rs933669421, rs933701929, rs934386655, rs934978163, rs935075927, rs935118943, rs935201380, rs935837682, rs935905673, rs936016464, rs936049453, rs936798914, rs936849720, rs937182249, rs937537909, rs937570665, rs937994813, rs938542544, rs938681632, rs938724654, rs938820223, rs940069091, rs940119854, rs940774368, rs940826687, rs941626852, rs941746676, rs941868729, rs941899907, rs941930940, rs942073217, rs942355726, rs942432222, rs942601151, rs942767965, rs942915660, rs943409756, rs943437575, rs943584392, rs944344709, rs944445504, rs944566226, rs944610754, rs944660235, rs945771980, rs945889685, rs945907716, rs945955008, rs946438576, rs946553780, rs946737049, rs946871622, rs946904307, rs947396402, rs947482304, rs947767605, rs948250636, rs948597868, rs948734301, rs949117362, rs949214481, rs950330868, rs950705348, rs950755485, rs950787024, rs950800408, rs950802707, rs951684989, rs951904607, rs951986741, rs952766100, rs953023418, rs953116152, rs953320098, rs953656604, rs954023902, rs954227184, rs954411504, rs955017973, rs955524334, rs956482948, rs956679571, rs957547658, rs957576262, rs957643960, rs958254598, rs958468993, rs958679276, rs958831944, rs959054760, rs959080048, rs959862424, rs960079378, rs960855187, rs961517950, rs961548849, rs961670956, rs962203011, rs962396646, rs962549970, rs963130955, rs963376639, rs963398510, rs963428122, rs964109866, rs964185666, rs964241634, rs964863374, rs965067483, rs965135657, rs965733835, rs965854041, rs965855346, rs965919402, rs966079231, rs966124914, rs967228454, rs967293775, rs967857394, rs968288418, rs968400786, rs968915078, rs968966373, rs969189680, rs969619610, rs969712289, rs969743528, rs970050628, rs970102964, rs970260156, rs970642706, rs970752785, rs971614757, rs971805380, rs971982192, rs972801487, rs972846148, rs972954085, rs972986813, rs973351599, rs973480210, rs973965482, rs974473340, rs974495259, rs974569731, rs975109224, rs975310606, rs975343022, rs975580984, rs976068531, rs976191178, rs976565240, rs976840004, rs977588208, rs977694596, rs977723343, rs978066013, rs978687520, rs978789840, rs978825750, rs979102783, rs979262079, rs979312963, rs980078924, rs980170235, rs980381352, rs980543716, rs980560543, rs980927881, rs981140991, rs981166844, rs981766622, rs982141722, rs982173901, rs982457001, rs982472219, rs982704303, rs983268531, rs983275242, rs983516728, rs983519579, rs983726579, rs984513793, rs985123065, rs985401039, rs985878756, rs986014626, rs986622472, rs986759200, rs986951798, rs987242196, rs987605510, rs988743954, rs988755435, rs989112401, rs989125004, rs989569450, rs989789326, rs990398094, rs990418442, rs990574734, rs991183945, rs991228363, rs991292577, rs991576110, rs991696356, rs991901021, rs991906459, rs992266417, rs992634606, rs992823492, rs992854512, rs992902742, rs993145340, rs993256498, rs993350747, rs993439960, rs993584607, rs993803707, rs995023471, rs995330923, rs995369226, rs995804858, rs995856828, rs995934514, rs996275539, rs996306987, rs996937371, rs997252470, rs997762316, rs997929337, rs998188482, rs998378447, rs999402044, rs999510527, rs999690646, rs999783145, rs1000474557, rs1000600182, rs1001071362, rs1001528360, rs1001608650, rs1001747555, rs1001951522, rs1002101646, rs1002170024, rs1002299249, rs1003532908, rs1003725150, rs1003750801, rs1004242162, rs1004271617, rs1004801952, rs1004911582, rs1005080791, rs1005741782, rs1005787891, rs1005880569, rs1005911736, rs1006115239, rs1006498313, rs1007433616, rs1007614007, rs1008436294, rs1008533458, rs1008680162, rs1009149007, rs1009983327, rs1009996976, rs1010281856, rs1010646277, rs1011412995, rs1011544775, rs1011602870, rs1011790910, rs1011821993, rs1011919840, rs1012301463, rs1012768862, rs1013351837, rs1013439564, rs1013456721, rs1013538206, rs1013736872, rs1014320721, rs1014391554, rs1014445538, rs1014487799, rs1014698874, rs1014766875, rs1014956181, rs1015402975, rs1015707461, rs1015987006, rs1016223617, rs1016338594, rs1016921910, rs1017210576, rs1017598132, rs1017904061, rs1018028868, rs1018216474, rs1018600315, rs1018745164, rs1018829555, rs1018901456, rs1019396693, rs1019465201, rs1019758173, rs1020091319, rs1020211135, rs1020285821, rs1020625905, rs1020814189, rs1021212529, rs1021512317, rs1021515202, rs1021894903, rs1022214013, rs1022756410, rs1023074800, rs1023227472, rs1023445348, rs1023596879, rs1023673292, rs1024560723, rs1024675303, rs1024750267, rs1024872134, rs1026118649, rs1026125205, rs1026173989, rs1026375519, rs1026432515, rs1026646691, rs1026959329, rs1027115730, rs1027232120, rs1027404148, rs1027691575, rs1028051042, rs1029183285, rs1029625611, rs1029808534, rs1029860898, rs1030058235, rs1030583170, rs1030816353, rs1031040164, rs1031072645, rs1031181284, rs1031768111, rs1031816555, rs1032075104, rs1032089551, rs1032238078, rs1032819150, rs1032850151, rs1033421195, rs1033502472, rs1034039219, rs1034314540, rs1034661154, rs1035401153, rs1035510107, rs1035599513, rs1035620253, rs1036068899, rs1036098665, rs1036540214, rs1036943567, rs1037134902, rs1037298336, rs1037523905, rs1037686914, rs1037737292, rs1037976441, rs1038768977, rs1038942252, rs1039189742, rs1039220774, rs1039308515, rs1039719927, rs1039773594, rs1040290756, rs1040356508, rs1040384067, rs1040484932, rs1041187153, rs1041690402, rs1041775405, rs1041799629, rs1042078798, rs1042694052, rs1042942141, rs1043436270, rs1043769887, rs1043811313, rs1043938202, rs1044465109, rs1044571174, rs1044623391, rs1045169491, rs1045313499, rs1046032232, rs1046176207, rs1046285797, rs1046569999, rs1046868119, rs1047668403, rs1047862660, rs1047993792, rs1048101751, rs1048621447, rs1049214066, rs1049478337, rs1049616911, rs1049710805, rs1050163422, rs1050193709, rs1050434515, rs1050476832, rs1050750972, rs1051171916, rs1051391979, rs1051796794, rs1052476761, rs1052940998, rs1053137488, rs1053366552, rs1053531092, rs1053561634, rs1053830662, rs1054088404, rs1054422480, rs1054537431, rs1054570667, rs1055129117, rs1055228042, rs1055325871, rs1055394925, rs1055693317, rs1055896493, rs1056115607, rs1056211073, rs1056554380, rs1056692237, rs1056995909, rs1057072923, rs1057231340, rs1057461074, rs1157789554, rs1157862088, rs1157873006, rs1157975194, rs1158032464, rs1158064716, rs1158116980, rs1158405815, rs1158746400, rs1158887915, rs1159262206, rs1159674512, rs1160006499, rs1160113335, rs1160600383, rs1160706797, rs1160865091, rs1160932397, rs1161035211, rs1161343096, rs1161347353, rs1161371056, rs1161414952, rs1161891218, rs1162362911, rs1162558724, rs1163000724, rs1163219125, rs1163367794, rs1163463017, rs1164368543, rs1164503069, rs1164847243, rs1164873912, rs1165008077, rs1165077265, rs1165336106, rs1165338154, rs1165773710, rs1165816738, rs1165976852, rs1166139101, rs1166421058, rs1166664326, rs1167139822, rs1167292125, rs1167348120, rs1167515102, rs1167631006, rs1168127730, rs1168264403, rs1168586459, rs1168710293, rs1168954386, rs1169301159, rs1169591561, rs1169603994, rs1169652198, rs1169658672, rs1170162097, rs1170390936, rs1170409666, rs1170942900, rs1170955780, rs1170998721, rs1171218356, rs1171702027, rs1172314889, rs1172434049, rs1172849541, rs1173707847, rs1173823751, rs1174814991, rs1175011392, rs1175327827, rs1175357608, rs1175617551, rs1176061395, rs1176082223, rs1176637215, rs1177205577, rs1177223675, rs1177247071, rs1177723770, rs1178058459, rs1178225126, rs1178319895, rs1178901661, rs1179112166, rs1180060955, rs1180090221, rs1180128343, rs1180384792, rs1180586601, rs1180979925, rs1181949601, rs1182016320, rs1182052395, rs1182203519, rs1182556058, rs1183149927, rs1183164326, rs1183235035, rs1183458275, rs1183656565, rs1183815600, rs1183867005, rs1184071025, rs1184100522, rs1184413545, rs1184673043, rs1185178481, rs1185182774, rs1185352145, rs1185478578, rs1185700026, rs1185871140, rs1186109181, rs1186314224, rs1186335669, rs1187038929, rs1187543280, rs1188310494, rs1188461427, rs1188512356, rs1188584042, rs1188947020, rs1189314261, rs1190008339, rs1190291765, rs1190318930, rs1190373636, rs1190446020, rs1190593443, rs1190812399, rs1191089627, rs1191135170, rs1191361066, rs1191672319, rs1191736100, rs1192085224, rs1192146980, rs1192524220, rs1192526344, rs1192900485, rs1193423937, rs1193517870, rs1193671728, rs1193936525, rs1194259344, rs1194863399, rs1194864484, rs1195347212, rs1195628635, rs1195653649, rs1196058953, rs1196143482, rs1196223578, rs1196238188, rs1196342785, rs1196434543, rs1196703055, rs1196755832, rs1196952517, rs1197025753, rs1197474485, rs1197607397, rs1197749558, rs1197807993, rs1197867913, rs1198100645, rs1198367904, rs1198410035, rs1198572675, rs1198578641, rs1198925032, rs1198984303, rs1199107235, rs1199222944, rs1199677544, rs1199753786, rs1200006955, rs1200097642, rs1200205022, rs1200450744, rs1201462711, rs1201740015, rs1201792281, rs1202447395, rs1202915332, rs1203299571, rs1203314353, rs1203603073, rs1203684628, rs1203735663, rs1203739845, rs1203868895, rs1204777833, rs1205198625, rs1205208786, rs1205611421, rs1205787125, rs1206158434, rs1206186754, rs1206356828, rs1206395079, rs1206476040, rs1206528577, rs1207023884, rs1207166504, rs1207517093, rs1207687198, rs1208016217, rs1208145886, rs1208592165, rs1208751986, rs1209478584, rs1209591053, rs1209615786, rs1209654588, rs1209727885, rs1210400997, rs1210627223, rs1210784337, rs1210807300, rs1210875333, rs1210991704, rs1211430430, rs1211907193, rs1212168895, rs1212466895, rs1212646625, rs1212911805, rs1213091841, rs1213260514, rs1213284856, rs1213343930, rs1213436006, rs1213523362, rs1213599528, rs1213721508, rs1213872268, rs1213888483, rs1213915250, rs1214676809, rs1214882799, rs1215508972, rs1216044176, rs1216124078, rs1216166145, rs1216196274, rs1216488525, rs1216775686, rs1216861048, rs1216984470, rs1217021289, rs1217067630, rs1218018911, rs1218038259, rs1218114280, rs1218527080, rs1218542557, rs1219287168, rs1219487594, rs1219511944, rs1219649459, rs1219862323, rs1219957648, rs1219966776, rs1220012165, rs1220244134, rs1220349343, rs1220412527, rs1220771333, rs1220961123, rs1221475710, rs1221649482, rs1221717703, rs1221862584, rs1221890852, rs1222542677, rs1222576908, rs1222690238, rs1222902554, rs1223091695, rs1223937763, rs1224385896, rs1224440486, rs1224736002, rs1224915381, rs1225058151, rs1225218606, rs1225653792, rs1225947216, rs1226111479, rs1226233633, rs1227244975, rs1227300436, rs1227518719, rs1227654520, rs1227757204, rs1228445306, rs1228618450, rs1228737361, rs1229051264, rs1229122926, rs1229187386, rs1229374529, rs1229470609, rs1229595302, rs1229787389, rs1230470699, rs1230486020, rs1230615078, rs1230892355, rs1231196218, rs1231206971, rs1231697581, rs1231743367, rs1231939060, rs1231996252, rs1232010060, rs1232315294, rs1232325085, rs1232576203, rs1233569147, rs1233946048, rs1234284026, rs1234690185, rs1234949027, rs1235351149, rs1235587143, rs1235810335, rs1235967629, rs1236003098, rs1236352225, rs1236391394, rs1236737229, rs1236794462, rs1236843542, rs1236870292, rs1237089108, rs1237122404, rs1237166830, rs1237707123, rs1237857178, rs1237948176, rs1238156061, rs1238261361, rs1238358177, rs1238359523, rs1238474945, rs1238522380, rs1238929942, rs1238971539, rs1239294080, rs1239637844, rs1239749655, rs1239906278, rs1240003631, rs1240519408, rs1240829858, rs1240899106, rs1241198225, rs1241931448, rs1242113837, rs1242430522, rs1242533462, rs1242763503, rs1243163393, rs1243267266, rs1243428097, rs1243472657, rs1243585873, rs1243862899, rs1244033403, rs1244173821, rs1244993746, rs1245201193, rs1245578738, rs1246931677, rs1247260531, rs1247816765, rs1248024678, rs1248060896, rs1248437949, rs1248544209, rs1248569986, rs1248802357, rs1249139797, rs1249322414, rs1249458980, rs1250155494, rs1250770757, rs1251016805, rs1251245881, rs1251326897, rs1251358440, rs1251758425, rs1251953671, rs1252106182, rs1252337801, rs1252495782, rs1252657358, rs1252660666, rs1252710277, rs1252880295, rs1253058595, rs1253077723, rs1253242497, rs1253497436, rs1253829650, rs1253877948, rs1254304551, rs1254456121, rs1254725722, rs1255170017, rs1255176233, rs1255585437, rs1255719236, rs1255807243, rs1255824540, rs1255879547, rs1256336865, rs1256462977, rs1256503383, rs1256636118, rs1256714520, rs1256764003, rs1256852735, rs1256955776, rs1257027033, rs1257035007, rs1257061512, rs1257133104, rs1257283181, rs1257820399, rs1258156203, rs1258273169, rs1258746292, rs1258960953, rs1259370327, rs1259383750, rs1259739412, rs1259932105, rs1260081702, rs1260313688, rs1260393510, rs1261083972, rs1261527305, rs1261850539, rs1262077799, rs1262098090, rs1262413926, rs1262544505, rs1262639959, rs1262900123, rs1263855981, rs1264202867, rs1264232614, rs1264283781, rs1264420948, rs1265270825, rs1265362443, rs1265959369, rs1266040631, rs1266131971, rs1266380289, rs1267088195, rs1267156543, rs1267255931, rs1267487067, rs1267951155, rs1268132758, rs1268246884, rs1268388401, rs1268395287, rs1268755351, rs1268977917, rs1269113483, rs1269541577, rs1269841792, rs1270093410, rs1270656193, rs1270830762, rs1270984077, rs1270996174, rs1271108275, rs1271284125, rs1271440684, rs1271703434, rs1272609267, rs1272647646, rs1272943322, rs1273410321, rs1273509015, rs1273919490, rs1274003511, rs1274207796, rs1274481893, rs1274542611, rs1274823884, rs1275743506, rs1275788168, rs1276075907, rs1276080275, rs1276415071, rs1276487800, rs1276869780, rs1277061706, rs1277327286, rs1277329140, rs1278472686, rs1278510099, rs1278666560, rs1279072825, rs1279369171, rs1279375726, rs1279398325, rs1279653359, rs1280034884, rs1280207817, rs1280830218, rs1280876755, rs1281197686, rs1281264913, rs1281288517, rs1281366733, rs1282550317, rs1282708695, rs1282747738, rs1282917218, rs1283117692, rs1283569564, rs1283672240, rs1284109108, rs1284297014, rs1284321540, rs1284674748, rs1284736585, rs1284746910, rs1285175021, rs1285643824, rs1285927544, rs1285959437, rs1286103737, rs1286282176, rs1286953472, rs1287951004, rs1288446045, rs1288778375, rs1288779598, rs1288978618, rs1289032716, rs1289145191, rs1289751873, rs1290477886, rs1290747656, rs1290798051, rs1291597427, rs1291804079, rs1292165059, rs1292274227, rs1292342639, rs1292825726, rs1292846797, rs1292848717, rs1293100682, rs1293145630, rs1293400908, rs1293593048, rs1293950126, rs1294315862, rs1294331112, rs1294353139, rs1294675670, rs1294698212, rs1294737986, rs1294777436, rs1294991620, rs1296387511, rs1296532201, rs1297111026, rs1297142569, rs1297300130, rs1297353859, rs1297457116, rs1297493767, rs1298410054, rs1299137065, rs1299628502, rs1300092127, rs1300321756, rs1300391510, rs1300467983, rs1300583545, rs1300990827, rs1300993082, rs1301032026, rs1301287267, rs1301812726, rs1302796260, rs1302901536, rs1302934844, rs1302975050, rs1303152256, rs1303177172, rs1303490856, rs1303541219, rs1303626084, rs1303936495, rs1304101529, rs1304152693, rs1305001261, rs1305087207, rs1305476831, rs1305503408, rs1305690011, rs1305705425, rs1305749094, rs1305915237, rs1307274498, rs1307330488, rs1308214684, rs1308394446, rs1308563802, rs1308655289, rs1309024654, rs1309044569, rs1309155156, rs1309350320, rs1310468302, rs1310734586, rs1310745905, rs1310868435, rs1311364650, rs1311440952, rs1311591202, rs1312099798, rs1312451353, rs1312569953, rs1312838349, rs1313367308, rs1313993605, rs1314112286, rs1314211719, rs1314302632, rs1314594608, rs1315335799, rs1315712310, rs1315934223, rs1315956121, rs1316121613, rs1316181063, rs1316366619, rs1316418126, rs1316967335, rs1316999338, rs1317598227, rs1317600548, rs1317608547, rs1317980432, rs1318081928, rs1318087994, rs1318153054, rs1318377769, rs1318378627, rs1318523628, rs1319045444, rs1320429541, rs1320525483, rs1321184316, rs1321353558, rs1321412256, rs1321591958, rs1321762134, rs1322004730, rs1322587716, rs1323159615, rs1323628531, rs1323891098, rs1323979463, rs1324023199, rs1324091818, rs1324408677, rs1324738210, rs1324824190, rs1324997654, rs1325095937, rs1325173246, rs1325194893, rs1325599006, rs1325617026, rs1326007980, rs1326226279, rs1326435237, rs1326698653, rs1326793710, rs1326915596, rs1327503571, rs1328042509, rs1328254096, rs1328413835, rs1328461083, rs1328591990, rs1329033271, rs1329334878, rs1329711161, rs1329847720, rs1330085321, rs1330134403, rs1330497806, rs1330912294, rs1331132952, rs1331383229, rs1331726183, rs1331927091, rs1332369196, rs1332462555, rs1332537504, rs1332542541, rs1333085275, rs1333223197, rs1333474492, rs1333691534, rs1334036133, rs1335228528, rs1335694215, rs1335742546, rs1335954460, rs1336062511, rs1336286510, rs1336427942, rs1336666013, rs1336900214, rs1336998932, rs1337209870, rs1337466582, rs1337496820, rs1337633458, rs1337646451, rs1337705064, rs1338049400, rs1338206940, rs1338365022, rs1338608410, rs1338683535, rs1338691421, rs1339012945, rs1339275716, rs1339863586, rs1340206299, rs1340305109, rs1340356988, rs1340544178, rs1340994569, rs1341009806, rs1341080071, rs1341467744, rs1341566724, rs1341882187, rs1341954374, rs1342133714, rs1342643601, rs1342751728, rs1342862561, rs1342926869, rs1343305941, rs1343691626, rs1343835677, rs1344104919, rs1344593596, rs1344675346, rs1344684878, rs1344737159, rs1344843522, rs1345044999, rs1345762309, rs1345771222, rs1345796501, rs1345918329, rs1345996094, rs1346182431, rs1346732399, rs1347045309, rs1347144366, rs1347192522, rs1347446912, rs1347648549, rs1348396551, rs1348552816, rs1348586250, rs1348665714, rs1349168408, rs1349362546, rs1349960067, rs1350152596, rs1350202479, rs1350219530, rs1350352333, rs1350419386, rs1350583452, rs1351200859, rs1351439088, rs1351714769, rs1351883279, rs1352059468, rs1352125157, rs1352159038, rs1352337103, rs1352390902, rs1352841243, rs1352871413, rs1354622435, rs1354882485, rs1355812331, rs1356221083, rs1356494411, rs1356505979, rs1356803161, rs1357079757, rs1357400031, rs1357718435, rs1357735556, rs1357857697, rs1358002965, rs1358345119, rs1358547535, rs1358568551, rs1359179018, rs1359257539, rs1359638704, rs1359670379, rs1359921525, rs1360283914, rs1360354727, rs1360408771, rs1361004331, rs1361024873, rs1361147632, rs1361224077, rs1361409445, rs1361515040, rs1361734366, rs1361878037, rs1361895403, rs1362351155, rs1362419332, rs1362670090, rs1362757815, rs1363009405, rs1363125434, rs1363154867, rs1363340910, rs1364018122, rs1364364689, rs1364914975, rs1365190336, rs1365844411, rs1365981777, rs1366147877, rs1366210809, rs1366522044, rs1367015188, rs1367276783, rs1367461510, rs1368010958, rs1368531338, rs1368545710, rs1368851499, rs1368938482, rs1369052226, rs1369117254, rs1369216633, rs1369562930, rs1369571737, rs1369653276, rs1369837786, rs1370309286, rs1370866508, rs1371238399, rs1371458310, rs1371695472, rs1371924109, rs1371941978, rs1371994605, rs1372196514, rs1372260778, rs1373338291, rs1373341039, rs1373402148, rs1373464819, rs1373497235, rs1374257700, rs1374399520, rs1374930651, rs1375789003, rs1376078690, rs1376661098, rs1376901094, rs1377048936, rs1377149111, rs1377219522, rs1377603775, rs1377653422, rs1377730582, rs1377861707, rs1377952272, rs1377981365, rs1378196373, rs1378355558, rs1378402354, rs1378425206, rs1378453746, rs1378585308, rs1379222175, rs1379680792, rs1380146979, rs1380281340, rs1380308782, rs1380673672, rs1380787962, rs1381046751, rs1381150645, rs1381488284, rs1381661694, rs1381981507, rs1382042533, rs1382068036, rs1382159395, rs1382386707, rs1382605471, rs1382729928, rs1382735305, rs1382949717, rs1383437155, rs1383497318, rs1383598333, rs1383617374, rs1383643837, rs1383713125, rs1383718261, rs1383918147, rs1384393182, rs1384708764, rs1386084723, rs1386122191, rs1386705076, rs1386722950, rs1387036676, rs1387040584, rs1387699457, rs1388138756, rs1388233437, rs1388516624, rs1388564312, rs1389297402, rs1389346008, rs1389407568, rs1389696309, rs1390096923, rs1390175132, rs1390283854, rs1390490344, rs1390608065, rs1390877375, rs1390939563, rs1391204216, rs1391596534, rs1391614732, rs1391692294, rs1391980514, rs1392032354, rs1392053850, rs1392267453, rs1392377776, rs1392442155, rs1392730067, rs1393116113, rs1393753209, rs1393905575, rs1393983726, rs1394158680, rs1394461711, rs1394470605, rs1394796637, rs1394837180, rs1394845003, rs1395104147, rs1395266743, rs1395291293, rs1395472834, rs1395727490, rs1396722127, rs1397149240, rs1397154270, rs1398239230, rs1398302322, rs1398487064, rs1398550306, rs1398954340, rs1399364087, rs1399740687, rs1399868299, rs1400618456, rs1400687913, rs1400794525, rs1400828843, rs1401029769, rs1401087488, rs1401548895, rs1401614480, rs1401809612, rs1401840464, rs1402402901, rs1402416475, rs1402505046, rs1402592281, rs1403033431, rs1403291922, rs1403683072, rs1403952333, rs1403953825, rs1404048074, rs1404509126, rs1404799039, rs1404933000, rs1404980858, rs1405172767, rs1405300182, rs1405321685, rs1405343326, rs1405422959, rs1405429189, rs1405434347, rs1405863463, rs1405928295, rs1406079339, rs1406090102, rs1406169924, rs1406239578, rs1406386084, rs1406447051, rs1406690516, rs1406735112, rs1407277972, rs1407344076, rs1407699225, rs1407756449, rs1407825133, rs1407910017, rs1408075612, rs1408144580, rs1408517295, rs1408751955, rs1409044250, rs1409122342, rs1409366208, rs1409384107, rs1409526716, rs1409854100, rs1410224642, rs1410888478, rs1411157801, rs1411359703, rs1411596187, rs1411615447, rs1411900924, rs1411989393, rs1412018354, rs1412648831, rs1413073784, rs1413304922, rs1413390523, rs1413453367, rs1413860255, rs1414011104, rs1414070551, rs1414547886, rs1414619735, rs1414621337, rs1414983255, rs1415002695, rs1415123666, rs1415231706, rs1415241524, rs1415314032, rs1415410741, rs1415414260, rs1415536649, rs1415987689, rs1416029611, rs1416035969, rs1416045715, rs1416107862, rs1416127881, rs1416419012, rs1416827028, rs1416858296, rs1417411740, rs1417622156, rs1417630602, rs1417818061, rs1417930950, rs1418791464, rs1418984580, rs1419204005, rs1419280412, rs1419713350, rs1419797420, rs1419893752, rs1420662454, rs1420688219, rs1420920188, rs1421289833, rs1421304911, rs1422081074, rs1422254670, rs1422739198, rs1423117655, rs1423118428, rs1423273299, rs1423367564, rs1423474105, rs1423544636, rs1423785497, rs1424116199, rs1424190194, rs1424191173, rs1424643590, rs1424974279, rs1425057940, rs1425080890, rs1425209751, rs1425339506, rs1425370132, rs1425536592, rs1425591308, rs1426708943, rs1427121726, rs1427413940, rs1427741494, rs1428197608, rs1428236851, rs1428357452, rs1428922283, rs1428999701, rs1429272861, rs1429368921, rs1429379530, rs1429424402, rs1429443039, rs1429447896, rs1430146671, rs1430241419, rs1430300164, rs1430333626, rs1430349119, rs1430405349, rs1430542250, rs1431087169, rs1431181701, rs1431276564, rs1431285172, rs1431646792, rs1431760548, rs1431858190, rs1432313427, rs1432861786, rs1433129743, rs1433220476, rs1433431800, rs1433893463, rs1434406529, rs1434854452, rs1434986142, rs1435160649, rs1435229830, rs1435235758, rs1435239484, rs1436135642, rs1436278952, rs1436388818, rs1436496034, rs1437254721, rs1437485597, rs1437812393, rs1438171619, rs1439462119, rs1439604150, rs1440078760, rs1440117271, rs1440149383, rs1440402063, rs1440597413, rs1440668026, rs1441329227, rs1442095488, rs1442573996, rs1442761181, rs1442813066, rs1443590425, rs1443741940, rs1444280167, rs1444480423, rs1444970726, rs1445347850, rs1445418428, rs1445681989, rs1446113684, rs1446279190, rs1446574901, rs1446784023, rs1447588065, rs1447861473, rs1448164511, rs1448271995, rs1448640930, rs1448668817, rs1448928691, rs1448986956, rs1449100808, rs1449359593, rs1449495110, rs1450048039, rs1450073278, rs1450929839, rs1451038771, rs1451108562, rs1451660485, rs1451729955, rs1451921740, rs1451936282, rs1452112797, rs1452353732, rs1452383285, rs1452809400, rs1453154691, rs1453484857, rs1454174495, rs1454494484, rs1454664690, rs1455007681, rs1455677374, rs1456028329, rs1456806383, rs1456877547, rs1457124899, rs1457237534, rs1457419182, rs1457588080, rs1457903189, rs1457903341, rs1458538521, rs1458959663, rs1459130664, rs1459197480, rs1459231954, rs1459425744, rs1459521631, rs1459760949, rs1460063687, rs1460686539, rs1460805562, rs1461154260, rs1461692220, rs1462343129, rs1462373662, rs1462732079, rs1462844011, rs1464612054, rs1465127871, rs1465354226, rs1465514880, rs1466059866, rs1466433314, rs1466854472, rs1466888045, rs1466950316, rs1467524245, rs1467777894, rs1468609921, rs1469200433, rs1469366141, rs1469499466, rs1469515809, rs1469521414, rs1470077505, rs1471208712, rs1471786116, rs1471817208, rs1471866095, rs1472115823, rs1472763613, rs1472887990, rs1472997376, rs1473202578, rs1473876543, rs1474230143, rs1474332469, rs1475049544, rs1475364442, rs1475368893, rs1476002826, rs1476223716, rs1476245490, rs1476447937, rs1476667379, rs1476957215, rs1477323673, rs1477410700, rs1477432905, rs1477876085, rs1477907482, rs1477923375, rs1478361217, rs1478602057, rs1478604397, rs1478782287, rs1479161072, rs1479494907, rs1479643037, rs1479891510, rs1480192399, rs1480222414, rs1480521244, rs1480811890, rs1480879620, rs1481226885, rs1481559015, rs1481599223, rs1482375880, rs1482482730, rs1482944438, rs1483371669, rs1483509821, rs1483762418, rs1484222730, rs1484232341, rs1484389923, rs1484479392, rs1484653137, rs1484691482, rs1485630690, rs1485657881, rs1485868938, rs1486164290, rs1486233446, rs1486344224, rs1486429340, rs1486458240, rs1486843371, rs1487438764, rs1487482586, rs1487685381, rs1487826125, rs1487937532, rs1487988407, rs1488291415, rs1488530469, rs1488810429, rs1489428172, rs1489508870, rs1489533316, rs1489606627, rs1489782171, rs1490307521, rs1490635561, rs1491324179 and rs1491360425.

It will be understood, however, that the diagnostic methods of the present disclosure may also be employed to detect other polymorphisms such as insertions and deletions. It will also be understood that polymorphisms located outside of TM6SF2 may also be employed in the presently disclosed method and still provide a diagnostic outcome. Such polymorphisms may, for example, be in linkage disequilibrium with a polymorphism located within TM6SF2.

Those skilled in the art will understand that a nucleic acid polymorphism in TM6SF2 can be detected using a variety of techniques. Some examples of suitable techniques include, but are not limited to, direct nucleic acid sequencing of TM6SF2, or a part thereof, capillary electrophoresis, hybridization of allele-specific probes or primers, single-strand conformation polymorphism analysis, denaturing gradient gel electrophoresis, temperature gradient electrophoresis, Sequenom MassARRAY, mismatch detection, nucleic acid arrays, primer specific extension, protein detection, and other techniques well known in the art. Other suitable techniques are described in WO2017/123696.

### Examples

### Sequences

Sequences relevant to the present examples are listed in Table 1.

**Table 1**

| **SEQ ID NO:** | **Description** | **Sequence** |
|---|---|---|
| 1 | Genomic region corresponding to TM6SF2 (sense) | See sequence listing. |
| | • start codon underlined | |
| 2 | Genomic region corresponding to TM6SF2 (antisense) | See sequence listing. |
| 3 | TM6SF2 mRNA | GCGCCCUGGAGAACGCGUCGGGGCUGAGCCGGGGUCCAGCAGCCGCCGCUAUGGACAUCC CGCCGCUGGCCGGCAAGAUCGCGGCGCUGUCGCUGAGCGCCCUCCCGGUGUCCUACGCGC UCAACCACGUCUCGGCGCUCUCGCACCCCCUGUGGGUGGCAUUGAUGAGCGCCCUAAUCC UGGGUCUGCUUUUCGUGGCGGUCUACAGCUUGUCCCAUGGCGAGGUCUCCUAUGACCCAC UCUAUGCUGUCUUCGCUGUCUUCGCCUUCACCUCGGUUGUGGACCUCAUCAUCGCUCUUC AGGAAGACAGCUAUGUGGUGGGCUUCAUGGAGUUCUACACCAAGGAGGGAGAGCCAUACC UGCGCACAGCGCACGGAGUCUUCAUCUGCUACUGGGAUGGCACUGUUCACUACCUCCUCU ACCUGGCCAUGGCCGGCGCCAUCUGCAGAAGGAAGAGAUACCGGAAUUUUGGACUCUACU GGCUGGGUUCCUUCGCCAUGAGCAUCCUGGUGUUCCUUACAGGAAACAUUCUUGGCAAAU ACAGCUCCGAGAUCAGGCCUGCCUUCUUCCUCACCAUCCCCUACCUGCUGGUGCCAUGCU GGGCUGGCAUGAAGGUCUUCAGCCAGCCCCGGGCGCUAACCCGCUGCACCGCCAACAUGG UGCAAGAGGAACAAAGAAAGGGACUCCUGCAGCGUCCGGCUGACCUGGCCCUUGUCAUAU AUCUCAUCCUUGCUGGCUUCUUCACUCUGUUCCGGGGCCUGGUGGUGCUUGAUUGCCCCA CAGAUGCCUGCUUUGUCUAUAUCUACCAGUAUGAGCCAUACCUGCGGGACCCUGUGGCCU ACCCUAAGGUGCAGAUGCUGAUGUACAUGUUUUAUGUCCUGCCUUUCUGCGGCCUGGCUG CCUAUGCUCUCACCUUCCCUGGUUGCUCCUGGCUUCCAGACUGGGCCUUGGUGUUUGCUG GAGGCAUCGGCCAGGCACAGUUCUCGCACAUGGGGGCUUCCAUGCACCUGCGCACACCCU UCACCUACCGUGUGCCUGAGGACACCUGGGGCUGCUUCUUCGUGUGCAAUCUGCUGUAUG CGCUGGGCCCCCACCUGCUGGCCUACCGUUGCCUUCAGUGGCCCGCAUUCUUCCACCAGC CACCACCCUCCGACCCCCUAGCCCUCCACAAGAAGCAGCAUUGAGAGAGCUGUGGACUCA GGACCCAGGACUCUGUUUACGUGCCCAGUCAGCCCUACCUGGGGAAGCGGGGGUUGGGUG UUUUAGAGACAGGAGGGACAUCUCCGGGUGUCUUUAGUCUUGGCUAUGGUAGUUUCAGUC CAGUGGGUGGGAUGGAGACCAACGACCAAGUGUUCCUGCAAGGAGAGCCUCCCACAGCUG UCACCAUGCUCCAACUCCCACACAAACGCUUCUACCCCUUCCAGAACUUACCCAUCAUCC CCCCAUGGAUCCUUUCAGUAAAAACCCUUUCAAUUUCCAAAAU |
| | • start codon underlined | |
| 4 | Antisense of TM6SF2 mRNA | AUUUUGGAAAUUGAAAGGGUUUUUACUGAAAGGAUCCAUGGGGGGAUGAUGGGUAAGUUC UGGAAGGGGUAGAAGCGUUUGUGUGGGAGUUGGAGCAUGGUGACAGCUGUGGGAGGCUCU CCUUGCAGGAACACUUGGUCGUUGGUCUCCAUCCCACCCACUGGACUGAAACUACCAUAG CCAAGACUAAAGACACCCGGAGAUGUCCCUCCUGUCUCUAAAACACCCAACCCCCGCUUC CCCAGGUAGGGCUGACUGGGCACGUAAACAGAGUCCUGGGUCCUGAGUCCACAGCUCUCU CAAUGCUGCUUCUUGUGGAGGGCUAGGGGGUCGGAGGGUGGUGGCUGGUGGAAGAAUGCG GGCCACUGAAGGCAACGGUAGGCCAGCAGGUGGGGGCCCAGCGCAUACAGCAGAUUGCAC ACGAAGAAGCAGCCCCAGGUGUCCUCAGGCACACGGUAGGUGAAGGGUGUGCGCAGGUGC AUGGAAGCCCCCAUGUGCGAGAACUGUGCCUGGCCGAUGCCUCCAGCAAACACCAAGGCC CAGUCUGGAAGCCAGGAGCAACCAGGGAAGGUGAGAGCAUAGGCAGCCAGGCCGCAGAAA GGCAGGACAUAAAACAUGUACAUCAGCAUCUGCACCUUAGGGUAGGCCACAGGGUCCCGC AGGUAUGGCUCAUACUGGUAGAUAUAGACAAAGCAGGCAUCUGUGGGGCAAUCAAGCACC ACCAGGCCCCGGAACAGAGUGAAGAAGCCAGCAAGGAUGAGAUAUAUGACAAGGGCCAGG UCAGCCGGACGCUGCAGGAGUCCCUUUCUUUGUUCCUCUUGCACCAUGUUGGCGGUGCAG CGGGUUAGCGCCCGGGGCUGGCUGAAGACCUUCAUGCCAGCCCAGCAUGGCACCAGCAGG UAGGGGAUGGUGAGGAAGAAGGCAGGCCUGAUCUCGGAGCUGUAUUUGCCAAGAAUGUUU CCUGUAAGGAACACCAGGAUGCUCAUGGCGAAGGAACCCAGCCAGUAGAGUCCAAAAUUC CGGUAUCUCUUCCUUCUGCAGAUGGCGCCGGCCAUGGCCAGGUAGAGGAGGUAGUGAACA GUGCCAUCCCAGUAGCAGAUGAAGACUCCGUGCGCUGUGCGCAGGUAUGGCUCUCCCUCC UUGGUGUAGAACUCCAUGAAGCCCACCACAUAGCUGUCUUCCUGAAGAGCGAUGAUGAGG UCCACAACCGAGGUGAAGGCGAAGACAGCGAAGACAGCAUAGAGUGGGUCAUAGGAGACC UCGCCAUGGGACAAGCUGUAGACCGCCACGAAAAGCAGACCCAGGAUUAGGGCGCUCAUC AAUGCCACCCACAGGGGGUGCGAGAGCGCCGAGACGUGGUUGAGCGCGUAGGACACCGGG AGGGCGCUCAGCGACAGCGCCGCGAUCUUGCCGGCCAGCGGCGGGAUGUCCAUAGCGGCG GCUGCUGGACCCCGGCUCAGCCCCGACGCGUUCUCCAGGGCGC |
| 5 | TM6SF2 siRNA #1 (sense) | CCUCGGUUGUGGACCUCAUUU |
| 6 | TM6SF2 siRNA #2 (sense) | GGAAGAGAUACCGGAAUUU |
| 7 | Non-targeting siRNA (control) | UAGCGACUAAACACAUCAA |

### Example 1

To determine whether HBV affects expression of TM6SF2, liver biopsies were taken from patients suffering from HBV infection and from healthy controls, and expression of TM6SF2 was measured in each group of subjects by quantitative PCR. As shown in Figure 1A, expression of TM6SF2 was significantly higher in HBV-infected subjects compared to non-infected controls (p < 0.0001).

TM6SF2 expression was also measured in a cell culture model. Specifically, Huh7 cells were transfected with HBV genotype D, and TM6SF2 expression was measured by quantitative PCR. As shown in Figure 1B, expression of TM6SF2 was significantly higher in HBV-infected cells compared to uninfected controls. Huh7 cells transfected with HBV genotype C also showed higher levels of TM6SF2 expression compared to uninfected controls (Figure 1C).

TM6SF2 expression was also measured in a mouse model. Specifically, liver samples were taken from mice infected with HBV (genotype A) and from non-infected controls, and expression of TM6SF2 was measured in each group of mice by quantitative PCR. As shown in Figure 1D, expression of TM6SF2 was significantly higher in HBV-infected mice compared to non-infected controls (p < 0.05).

Analysis of the GEO dataset GDS2339 also demonstrated that expression of TM6SF2 was higher in HepG2 human hepatoma cells expressing HBsAg compared to wild type controls (Figure 1E). TM6SF2 induction was specific as expression of the lipid-related gene PNPLA3 was not increased by HBV infection *in vitro,* and PNPLA3 expression did not correlate with HBV viral load.

### Example 2

A small interfering RNA (siRNA) was designed to target TM6SF2 and silence its expression by RNAi (SEQ ID NO. 5 and SEQ ID NO. 6). Huh7 cells infected with HBV (genotype D) were treated with the TM6SF2-specific siRNA or a scramble control, and the level of HBsAg in the supernatant was measured. Enzyme-linked immunosorbent assay (ELISA) demonstrated that inhibiting TM6SF2 (by approximately 80%) significantly reduced the amount of HBsAg secreted by the cells (Figure 2A; p < 0.0001). Western blotting also confirmed that inhibition of TM6SF2 reduced the levels of HBsAg in the supernatant and cell lysates (Figure 2B). Inhibition of TM6SF2 also reduced the amount of HBsAg (genotype C) in Huh7 cells (Figure 2C).

HepaRG cells (differentiated primary hepatocytes) infected with HBV (genotype D) were treated with the TM6SF2-specific siRNA (SEQ ID NO: 6) or a scramble control, and the level of HBsAg in the supernatant was measured. ELISA demonstrated that inhibiting TM6SF2 significantly reduced the amount of HBsAg secreted by the cells (Figure 2D).

HBsAg levels were then measured in blood (serum) samples taken from 65 patients suffering from chronic HBV. The patients were grouped based on their TM6SF2 genotype, and in particular, based on the presence or absence of the rs58542926 polymorphism (C → T substitution), which encodes an E167K amino acid substitution, and reduces TM6SF2 levels in the liver. As shown in Figure 2E, approximately 25% less HBsAg was detected in patients carrying the CT/TT genotype compared to patients carrying the CC genotype (p < 0.05) (n: CC = 42, CT = 21, TT = 2).

### Example 3

To examine the effect of HBV infection and TM6SF2 expression on intracellular lipid content *in vitro,* cytoplasmic lipid droplets in Huh7 cells were labelled with fluorescent BODIPY 558/568 C₁₂ (4,4-difluoro-5-(2-thienyl)-4-bora-3a,4a-diaza-s-indacene-3-dodecanoic acid) (Boulant et al. (2008) Traffic 9: 1268-1282). Inhibition of TM6SF2 with a siRNA (SEQ ID NO. 6) led to an approximately 6-fold increase in lipid droplet area and an approximately 12-fold increase in size (Figure 3B) compared to cells treated with a scramble control (Figure 3A). Although cells infected with HBV (Figure 3C) had similar cellular lipid to mock infected cells (Figure 3A), reducing TM6SF2 in HBV infected cells did not increase lipid (Figure 3D).

### Example 4

An *in vitro* model of HDV/HBV co-infection was established by transfecting Huh7 cells with DNA plasmids encoding HBV and HDV (Figure 4A). Co-infection with HBV and HDV induced TM6SF2 expression (Figure 4B; p < 0.0001) more than HBV or HDV alone (p < 0.01). Cells were then treated with a siRNA targeting TM6SF2 (SEQ ID NO. 6) or a scramble control. Inhibition of TM6SF2 reduced HDV RNA in cell lysates (Figure 4C; p < 0.001) and reduced HBsAg secretion (Figure 4D; p < 0.01) in supernatant and cell lysate (Figure 4E).

### Example 5

Huh7 cells infected with HBV genotype C and HBV genotype D were treated with TM6SF2-specific siRNA (SEQ ID NO. 5 and SEQ ID NO. 6) or a scramble control. As shown in Figure 5, inhibition of TM6SF2 caused a significant reduction in HBV cccDNA levels for both genotypes.

### Example 6

To further explore the role of TM6SF2 expression in HBV infection, the CRISPR/Cas9 editing system was employed to delete a 4.5 kbp region of the TM6SF2 gene, using dual guide RNAs targeting both exons 1 and 6 of the gene (Figure 6A). This approach achieved >99% reduction of TM6SF2 expression in Huh7 cells (Figure 6B; p<0.001), while maintaining cell viability. Reduced HBs-Ag secretion is expected (not shown).

## Claims

1. An inhibitor of transmembrane 6 superfamily member 2 (TM6SF2) for use in a method of treating a hepatitis B and/or hepatitis D virus infection in a subject, the method comprising administering to the subject said inhibitor, wherein the inhibitor is a nucleic acid or a small organic molecule.

2. The inhibitor for use of claim 1, wherein the inhibitor is a nucleic acid.

3. The inhibitor for useof claim 1 or claim 2, wherein the nucleic acid comprises at least 20 contiguous nucleotides which are substantially complementary to the nucleotide sequence set forth in SEQ ID NO. 3.

4. The inhibitor for use of any one of claims 1 to 3, wherein the nucleic acid comprises at least 10 contiguous nucleotides differing by no more than 3 nucleotides from a nucleotide sequence set forth in any one of SEQ ID NOs. 1, 2 or 4.

5. The inhibitor for use of any one of claims 1 to 4, wherein the nucleic acid comprises at least 10 contiguous nucleotides differing by no more than 3 nucleotides from a nucleotide sequence set forth in SEQ ID NO. 4, optionally wherein the nucleic acid comprises at least 20 contiguous nucleotides differing by no more than 3 nucleotides from a nucleotide sequence set forth in SEQ ID NO. 4.

6. The inhibitor for use of any one of claims 1 to 5, wherein the nucleic acid comprises at least 10 contiguous nucleotides which are at least 80% identical to a nucleotide sequence set forth in any one of SEQ ID NOs 1, 2 or 4.

7. The inhibitor for use of any one of claims 1 to 6, wherein the nucleic acid comprises at least 10 contiguous nucleotides which are at least 80% identical to a nucleotide sequence set forth in SEQ ID NO 4.

8. The inhibitor for use of any one of claims 1 to 7, wherein the nucleic acid comprises at least 20 contiguous nucleotides which are at least 80% identical to a nucleotide sequence set forth in SEQ ID NO 4.

9. The inhibitor for use of any one of claims 1 to 8, wherein the nucleic acid comprises the sequence set forth in SEQ ID NO. 5 or SEQ ID NO. 6 and a sequence substantially complementary thereto.

10. The inhibitor for use of claim 9, wherein the nucleic acid is between 15 and 50 nucleotides in length.

11. The inhibitor for use of any one of claims 1 to 10, wherein the nucleic acid is a siRNA or a nucleic acid encoding a siRNA, optionally wherein the siRNA is conjugated to N-acetylgalactosamine.

12. The inhibitor for use of any one of claims 1 to 11, wherein the method further comprises administering to the subject an antiviral agent, optionally wherein the antiviral agent is an interferon or a nucleoside analogue, optionally wherein the interferon is pegylated interferon.

13. The inhibitor for use of claim 12, wherein the antiviral agent and the inhibitor are administered to the subject in separate compositions, optionally wherein the antiviral agent and the inhibitor are administered to the subject sequentially or simultaneously.

14. The inhibitor for use of any one of the preceding claims, wherein the subject is suffering from acute hepatitis B virus infection, or wherein the subject is suffering from chronic hepatitis B virus infection.

15. The inhibitor for use of any one of claims 1 to 14, wherein the subject is a human.

## Patentansprüche

1. Inhibitor des Transmembran-6-Superfamilienmitglieds 2 (TM6SF2) zur Verwendung in einem Verfahren zum Behandeln einer Hepatitis B- und/oder Hepatitis D-Virusinfektion eines Subjekt, das Verfahren umfassend das Verabreichen des Inhibitors an das Subjekt, wobei der Inhibitor eine Nukleinsäure oder ein kleines organisches Molekül ist.

2. Inhibitor zur Verwendung nach Anspruch 1, wobei der Inhibitor eine Nukleinsäure ist.

3. Inhibitor zur Verwendung nach Anspruch 1 oder 2, wobei die Nukleinsäure mindestens 20 aufeinanderfolgende Nukleotide umfasst, die im Wesentlichen komplementär zu der Nukleotidsequenz sind, die in SEQ ID NO. 3 angegeben ist.

4. Inhibitor zur Verwendung nach einem der Ansprüche 1 bis 3, wobei die Nukleinsäure mindestens 10 aufeinanderfolgende Nukleotide umfasst, die sich um nicht mehr als 3 Nukleotide von einer Nukleotidsequenz unterscheiden, die in einer der SEQ ID NOs 1, 2 oder 4 angegeben ist.

5. Inhibitor zur Verwendung nach einem der Ansprüche 1 bis 4, wobei die Nukleinsäure mindestens 10 aufeinanderfolgende Nukleotide umfasst, die sich um nicht mehr als 3 Nukleotide von einer Nukleotidsequenz unterscheiden, die in SEQ ID NO. 4 angegeben ist, optional wobei die Nukleinsäure mindestens 20 aufeinanderfolgende Nukleotide umfasst, die sich um nicht mehr als 3 Nukleotide von einer Nukleotidsequenz unterscheiden, die in SEQ ID NO. 4 angegeben ist.

6. Inhibitor zur Verwendung nach einem der Ansprüche 1 bis 5, wobei die Nukleinsäure mindestens 10 aufeinanderfolgende Nukleotide umfasst, die mindestens zu 80 % mit einer Nukleotidsequenz identisch sind, die in einer der SEQ ID NOs 1, 2 oder 4 angegeben ist.

7. Inhibitor zur Verwendung nach einem der Ansprüche 1 bis 6, wobei die Nukleinsäure mindestens 10 aufeinanderfolgende Nukleotide umfasst, die mindestens zu 80 % mit einer Nukleotidsequenz identisch sind, die in SEQ ID NO. 4 angegeben ist.

8. Inhibitor zur Verwendung nach einem der Ansprüche 1 bis 7, wobei die Nukleinsäure mindestens 20 aufeinanderfolgende Nukleotide umfasst, die mindestens zu 80 % mit einer Nukleotidsequenz identisch sind, die in SEQ ID NO. 4 angegeben ist.

9. Inhibitor zur Verwendung nach einem der Ansprüche 1 bis 8, wobei die Nukleinsäure die Sequenz, die in SEQ ID NO. 5 oder SEQ ID NO. 6 angegeben ist, und eine Sequenz umfasst, die dazu im Wesentlichen komplementär ist.

10. Inhibitor zur Verwendung nach Anspruch 9, wobei die Nukleinsäure zwischen 15 und 50 Nukleotide lang ist.

11. Inhibitor zur Verwendung nach einem der Ansprüche 1 bis 10, wobei die Nukleinsäure eine siRNA oder eine Nukleinsäure ist, die eine siRNA codiert, optional wobei die siRNA an N-Acetylgalactosamin konjugiert ist.

12. Inhibitor zur Verwendung nach einem der Ansprüche 1 bis 11, wobei das Verfahren ferner das Verabreichen eines antiviralen Mittels an das Subjekt umfasst, optional wobei das antivirale Mittel ein Interferon oder ein Nukleosid-Analogon ist, wobei das Interferon optional pegyliertes Interferon ist.

13. Inhibitor zur Verwendung nach Anspruch 12, wobei das antivirale Mittel und der Inhibitor dem Subjekt in getrennten Zusammensetzungen verabreicht werden, optional wobei das antivirale Mittel und der Inhibitor dem Subjekt nacheinander oder gleichzeitig verabreicht werden.

14. Inhibitor zur Verwendung nach einem der vorstehenden Ansprüche, wobei das Subjekt an einer akuten Hepatitis B-Virusinfektion leidet, oder wobei das Subjekt an einer chronischen Hepatitis B-Virusinfektion leidet.

15. Inhibitor zur Verwendung nach einem der Ansprüche 1 bis 14, wobei das Subjekt ein Mensch ist.

## Revendications

1. Inhibiteur du membre 2 de la superfamille de la transmembrane 6 (TM6SF2) pour une utilisation dans une méthode de traitement d'une infection par le virus de l'hépatite B et/ou de l'hépatite D chez un sujet, la méthode comprenant l'administration dudit inhibiteur au sujet, dans lequel l'inhibiteur est un acide nucléique ou une petite molécule organique.

2. Inhibiteur pour une utilisation selon la revendication 1, dans lequel l'inhibiteur est un acide nucléique.

3. Inhibiteur pour une utilisation selon la revendication 1 ou la revendication 2, dans lequel l'acide nucléique comprend au moins 20 nucléotides contigus qui sont sensiblement complémentaires de la séquence de nucléotides présentée dans SEQ ID NO. 3.

4. Inhibiteur pour une utilisation selon l'une quelconque des revendications 1 à 3, dans lequel l'acide nucléique comprend au moins 10 nucléotides contigus ne différant pas de plus de 3 nucléotides d'une séquence de nucléotides présentée dans l'une des séquences SEQ ID NO. 1, 2 ou 4.

5. Inhibiteur pour une utilisation selon l'une quelconque des revendications 1 à 4, dans lequel l'acide nucléique comprend au moins 10 nucléotides contigus ne différant pas de plus de 3 nucléotides d'une séquence de nucléotides présentée dans SEQ ID NO. 4, éventuellement dans lequel l'acide nucléique comprend au moins 20 nucléotides contigus ne différant pas de plus de 3 nucléotides d'une séquence de nucléotides présentée dans SEQ ID NO. 4.

6. Inhibiteur pour une utilisation selon l'une quelconque des revendications 1 à 5, dans lequel l'acide nucléique comprend au moins 10 nucléotides contigus qui sont au moins 80 % identiques à une séquence de nucléotides présentée dans l'une quelconque des SEQ ID NO 1, 2 ou 4.

7. Inhibiteur pour une utilisation selon l'une quelconque des revendications 1 à 6, dans lequel l'acide nucléique comprend au moins 10 nucléotides contigus qui sont au moins 80 % identiques à une séquence de nucléotides présentée dans SEQ ID NO 4.

8. Inhibiteur pour une utilisation selon l'une quelconque des revendications 1 à 7, dans lequel l'acide nucléique comprend au moins 20 nucléotides contigus qui sont au moins 80 % identiques à une séquence de nucléotides présentée dans SEQ ID NO 4.

9. Inhibiteur pour une utilisation selon l'une quelconque des revendications 1 à 8, dans lequel l'acide nucléique comprend la séquence présentée dans SEQ ID NO. 5 ou SEQ ID NO. 6 et une séquence qui lui est sensiblement complémentaire.

10. Inhibiteur pour une utilisation selon la revendication 9, dans lequel l'acide nucléique a une longueur comprise entre 15 et 50 nucléotides.

11. Inhibiteur pour une utilisation selon l'une quelconque des revendications 1 à 10, dans lequel l'acide nucléique est un ARN interférent court ou un acide nucléique codant pour un ARN interférent court, éventuellement dans lequel l'ARN interférent court est conjugué à N-acétylgalactosamine.

12. Inhibiteur pour une utilisation selon l'une quelconque des revendications 1 à 11, dans lequel la méthode comprend en outre l'administration au sujet d'un agent antiviral, éventuellement dans lequel l'agent antiviral est un interféron ou un analogue de nucléoside, éventuellement dans lequel l'interféron est un interféron pégylé.

13. Inhibiteur pour une utilisation selon la revendication 12, dans lequel l'agent antiviral et l'inhibiteur sont administrés au sujet dans des compositions séparées, éventuellement dans lequel l'agent antiviral et l'inhibiteur sont administrés au sujet de manière séquentielle ou simultanée.

14. Inhibiteur pour une utilisation selon l'une quelconque des revendications précédentes, dans lequel le sujet souffre d'une infection aiguë par le virus de l'hépatite B, ou dans lequel le sujet souffre d'une infection chronique par le virus de l'hépatite B.

15. Inhibiteur pour une utilisation selon l'une quelconque des revendications 1 à 14, dans lequel le sujet est un humain.
